(19) Europäisches Patentamt
European Patent Office
Office européen des brevets

(11) Veröffentlichungsnummer: **0 085 275**
**B1**

(12) # EUROPÄISCHE PATENTSCHRIFT

(45) Veröffentlichungstag der Patentschrift: **19.02.86**

(51) Int. Cl.[4]: **C 07 D 277/54,** C 07 F 9/65, A 61 K 31/425

(21) Anmeldenummer: **82810418.2**

(22) Anmeldetag: **08.10.82**

(54) **Substituierte Thiazolidinylester von Mineralsäuren.**

(30) Priorität: **22.01.82 CH 406/82**

(43) Veröffentlichungstag der Anmeldung: **10.08.83 Patentblatt 83/32**

(45) Bekanntmachung des Hinweises auf die Patenterteilung: **19.02.86 Patentblatt 86/8**

(84) Benannte Vertragsstaaten: **AT BE CH DE FR IT LI LU NL SE**

(56) Entgegenhaltungen:
**DE - A - 2 405 395**
**DE - A - 2 632 746**
**DE - A - 3 002 733**

***Die Akte enthält technische Angaben, die nach dem Eingang der Anmeldung eingereicht wurden und die nicht in dieser Patentschrift enthalten sind.***

(73) Patentinhaber: **CIBA-GEIGY AG, Klybeckstrasse 141, CH-4002 Basel (CH)**

(72) Erfinder: **Storni, Angelo, Dr., Im Feuerbusch 3, CH-4310 Rheinfelden (CH)**

**EP 0 085 275 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung sind neue substituierte Thiazolidinylester von Mineralsäuren und Salze solcher Verbindungen mit wertvollen pharmakologischen Eigenschaften, Verfahren zur Herstellung dieser neuen Stoffe, sowie pharmazeutische Präparate, welche diese Stoffe enthalten.

In der Deutschen Offenlegungsschrift 2 405 395 sind unter anderem Verbindungen der allgemeinen Formel

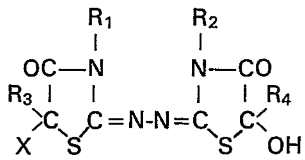

beschrieben, worin eines der Symbole $R_1$ und $R_2$ Allyl, 1-Methylallyl, 2-Methylallyl oder 2-Propinyl und das andere ebenfalls eine dieser Gruppen oder Methyl, $R_3$ und $R_4$ unabhängig voneinander Wasserstoff oder Methyl und X Wasserstoff oder Hydroxy bedeuten. Diese Verbindungen hemmen das Wachstum von Tumoren.

Eine Aufgabe der vorliegenden Erfindung ist, neue Verbindungen bereitzustellen, die gegenüber dem oben beschriebenen Stand der Technik verbesserte pharmakologische Eigenschaften aufweisen.

Die erfindungsgemässen Verbindungen entsprechen der allgemeinen Formel

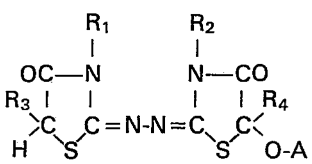 (I)

in welcher eines der Symbole $R_1$ und $R_2$ einen in der 2,3-Stellung ungesättigten Alkylrest mit 3 od. 4 Kohlenstoffatomen und das andere einen solchen Rest oder Niederalkyl mit bis 7 Kohlenstoffatomen bedeuten, $R_3$ und $R_4$ unabhängig voneinander Wasserstoff oder Methyl darstellen, und A einen Rest der Formel

$-SO_2-O-Z_1$ oder 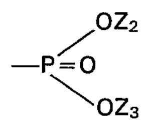

(Ia) (Ib)

bedeutet, in welchem $Z_1$ bzw. $Z_2$ und $Z_3$ unabhängig voneinander für Wasserstoff oder Niederalkyl mit bis 7 Kohlenstoffatomen stehen, oder $Z_2$ und $Z_3$ zusammen Niederalkylen mit 2 bis 5 Kohlenstoffatomen bedeuten. Ebenfalls Gegenstand der Erfindung sind die Salze von Verbindungen der allgemeinen Formel I, in denen $Z_1$ bzw. $Z_3$ und gegebenenfalls auch $Z_2$ Wasserstoff bedeuten, mit Basen, insbesondere die pharmazeutisch annehmbaren Salze mit Basen.

In den Verbindungen der allgemeinen Formel I enthält ein in der 2,3-Stellung ungesättigter Alkylrest $R_1$ und/oder $R_2$ eine Doppel- oder Dreifachbindung und stellt z.B. Allyl, 1- oder 2-Methylallyl oder 2-Propinyl dar. Ein Niederalkylrest $R_1$ bzw. $R_2$, sowie gegebenenfalls als $Z_1$ bzw. als $Z_2$ und $Z_3$ vorliegendes Niederalkyl enthält bis 7 und vorzugsweise bis 4 Kohlenstoffatome und ist beispielsweise Pentyl, Isopentyl, Neopentyl, Hexyl, Heptyl, vorzugsweise Propyl, Isopropyl, Butyl, Isobutyl, vor allem aber Äthyl oder in erster Linie Methyl. Durch $Z_2$ und $Z_3$ zusammen gebildetes Niederalkylen weist 2 bis 5 Kohlenstoffatome mit 3 oder vorzugsweise 2 Kettengliedern auf und ist z.B. Propylen, 1,2-Dimethyläthylen, Trimethylen, 2-Methyltrimethylen, 1,3- oder 2,2-Dimethyltrimethylen oder vor allem Äthylen.

Salze mit Basen von zur Salzbildung fähigen Verbindungen der allgemeinen Formel I sind z.B. Metall-, wie Alkalimetall-, z.B. Natrium- oder Kaliumsalze, oder Erdalkalimetall-, wie Magnesium- oder Calciumsalze, ferner Ammoniumsalze und Salze mit primären, sekundären oder tertiären einsäurigen oder mehrsäurigen organischen Basen, wie z.B. mit Äthylamin, 2-Aminoäthanol, Diäthylamin, Iminodiäthanol, Triäthylamin, 2-(Diäthylamino)-äthanol, Nitrilotriäthanol oder Pyridin, bzw. 1,2-Äthandiamin. Bevorzugt sind die entsprechenden pharmazeutisch verwendbaren, nicht-toxischen Salze.

Verbindungen der allgemeinen Formel I mit einem Rest A der Teilformel Ib können entsprechend der Definition von $Z_2$ und $Z_3$ sowohl neutrale Phosphorsäureester, als auch saure, d.h. einbasige (mit $Z_2$ als Niederalkyl und $Z_3$ als Wasserstoff) oder zweibasige (mit $Z_2$ und $Z_3$ als Wasserstoff), Phosphorsäureester sein.

Die Verbindungen der Formel I können in Form von Isomerengemischen, z.B. Gemischen von Racematen (Diastereomerengemisch) oder Racematen, oder in Form von reinen Isomeren, z.B. reinen Racematen bzw. optischen Antipoden vorliegen.

Die neuen Verbindungen der allgemeinen Formel I und die Salze solcher Verbindungen besitzen wertvolle pharmakologische Eigenschaften, insbesondere Tumor-hemmende Wirksamkeit. Diese kann im Tierversuch, z.B. bei oraler oder parenteraler, wie intraperitonealer oder subcutaner Verabreichung von Dosen zwischen 10 und 250 mg/kg am Ehrlich Karzinom der Maus [Transplantat: 1 × $10^6$ Zellen (Ascites) i.p. an weibliche Mäuse NMRI], am Walker Karzinosarkom 256 der Ratte [Transplantat: 0,5 ml einer Suspension von soliden Tumoren in Hanks'-Lösung s.c. oder i.m. an männlichen Ratten (Wistar)], am transplantablen Mamma-Adenokarzinom R 3230 AC der Ratte [Transplantat: 0,5 ml einer Suspension von soliden Tumoren in Hanks'-Lösung s.c. oder i.m. an weiblichen Ratten (Fischer)], und besonders am durch 7,12 Dimethylbenz[α]anthracen (DMBA) induzierten Mammakarzinom der Ratte (induziert durch p.o. Verabreichung von 15 mg DMBA in 1 ml Sesamöl an 50 Tage alte weibliche Ratten (Sprague Dawley), wobei multiple Tumoren nach 6 bis 8 Wochen festgestellt werden können).

So kann z.B. beim Ehrlich Karzinom nach viermaliger intraperionealer Verabreichung (4 Stunden nach, dann 1, 2 und 3 Tage nach der Transplantation; 10 Tiere pro Dosis; 10 Tage nach der Transplantation wird die Ascitesmenge in ml bestimmt), beim Walker Karzinosarkom 256 nach viermaliger oraler oder intraperitonealer Verabreichung (1, 2, 3 und 4 Tage nach Transplantation; 8 bis 10 Tiere/Dosis; 10 Tage

nach der Transplantation wird das Tumorgewicht in g bestimmt), und beim Mamma-Adenokarzinom R 3230 AC nach 10maliger oraler oder intraperitonealer Verabreichung (5 × pro Woche während 2 Wochen mit Beginn 4 Stunden nach Transplantation; 10 bis 15 Tiere/Dosis; 20 Tage nach der Transplantation wird das Tumorgewicht in g bestimmt) folgende Hemmung des Tumorwachstums im Vergleich mit unbehandelten Kontrolltieren festgestellt werden:

| Verbindung (Beispiel) | Ehrlich Ascites Karzinom | | Walker Karzinosarkom 256 | | Mamma-Adenokarzinom R 3230 Ac | |
|---|---|---|---|---|---|---|
| | Dosis (mg/kg) | Hemmung des Tumorwachstums (in %) | Dosis (mg/kg) | Hemmung des Tumorwachstums (in %) | Dosis (mg/kg) | Hemmung des Tumorwachstums (in %) |
| 1 | 4×50 i.p. | 94 | 4×50 i.p.<br>4×250 p.o. | 84<br>53 | 10×50 i.p.<br>10×250 p.o. | 62<br>43 |
| 5 | 4×50 i.p. | 42 | 4×50 p.o. | 72 | — | — |
| 6 | 4:100 i.p. | 70 | 4×100 i.p. | 53 | — | — |
| 7 | 4×100 i.p. | 51 | 4×50 i.p.<br>4×250 p.o. | 31<br>32 | —<br>— | —<br>— |
| 10 | 4×50 i.p. | 21 | — | — | — | — |
| 11 | 4×50 i.p. | 49 | — | — | — | — |

(Verabreichungsart i.p.: intraperitoneal, und p.o.: oral)

Beim DMBA-induzierten Mammakarzinom kann folgende Hemmung des Tumorwachstums und Rückbildung von Tumoren nach fünfwöchiger (25 Einzeldosen) bzw. sechswöchiger (30 Einzeldosen) Behandlung festgestellt werden (die angegebenen Zahlen zeigen die durchschnittliche Grösse aller Tumoren in sämtlichen Versuchstieren):

| Verbindung (Beispiel) | Dosis mg/kg | durschnittliche Tumorgrösse (behandelte/ unbehandelte Versuchstiere[a]) | Tumorreduktion (in Prozenten) |
|---|---|---|---|
| 1 | 30×10 s.c.<br>30×25 p.o. | 1,13/24,73<br>2,50/20,63 | 95<br>88 |
| 5 | 25×25 i.p.<br>25×100 p.o. | 6,31/15,97<br>0,96/19,03 | 61<br>95 |
| 6 | 25×100 p.o. | 2,13/21,55 | 90 |
| 7 | 30×10 s.c.<br>30×25 p.o. | 2,27/24,82<br>6,38/21,04 | 91<br>70 |
| 10 | 30×10 s.c.<br>30×25 p.o. | 8,77/22,27<br>6,07/22,27 | 61<br>72 |
| 11 | 30×10 s.c.<br>30×25 p.o. | 9,03/22,27<br>5,65/22,27 | 59<br>75 |

(Verabreichungsart s.c.: subcutan; p.o.: per oral; i.p.: intraperitoneal; (a): die angegebenen Zahlen zeigen die durchschnittliche Grösse aller Tumoren in sämtlichen Versuchstieren).

Im Vergleich zur starken Antitumor-Wirksamkeit sind die Toxizität und die Nebenwirkungen der erfindungsgemässen Verbindungen gering bis mässig (einmalige, maximal tolerierte Dosis: intraperitoneal zwischen 500 und 1250 mg/kg, und per oral grösser als 2500 mg/kg), so dass sie als solche oder insbesondere in Form von pharmazeutischen Präparaten zur Behandlung von neoplastischen Krankheiten bei Warmblütern durch enterale, insbesondere orale, oder parenterale Verabreichung von therapeutisch wirksamen Dosen, insbesondere zur Behandlung des Mammacarcinoms, Verwendung finden können.

Die Erfindung betrifft insbesondere solche Verbindungen der allgemeinen Formel I, in welchen einer der Reste $R_1$ und $R_2$ Allyl oder 2-Methylallyl und der andere ebenfalls eine dieser Gruppen oder vorzugsweise Methyl bedeuten, während $R_3$, $R_4$ und A die unter der Formel I angegebenen Bedeutungen haben können, A jedoch insbesondere ein Rest der Teilformel Ia mit Wasserstoff als $Z_1$, oder vorzugsweise ein Rest der Teilformel Ib mit Niederalkyl, vor allem Methyl als $Z_2$, und Niederalkyl, vor allem Methyl, oder Wasserstoff als $Z_3$ ist, und Salze, insbesondere pharmazeutisch annehmbare Salze mit Basen von solchen Verbindungen, in denen $Z_1$ bzw. $Z_3$ und gegebenenfalls $Z_2$ Wasserstoff bedeuten, z.B. die entsprechenden Alkalimetall-, wie die Natriumsalze.

Vor allem betrifft die Erfindung Verbindungen der Formel I, in denen $R_1$ Allyl oder 2-Methylallyl und $R_2$ ebenfalls einer dieser Reste oder vorzugsweise Methyl sind, $R_3$ Wasserstoff oder insbesondere Methyl und $R_4$ Wasserstoff ist, während A die unter der Formel I angegebene Bedeutung, jedoch in erster Linie die vorstehend angegebenen bevorzugten Bedeutungen hat, wobei im Rest der Formel Ib $Z_2$ insbesondere Methyl und $Z_3$ insbesondere Methyl oder Wasserstoff ist, und Salze, insbesondere pharmazeutisch verwendbare Salze von solchen Verbindungen, in denen $Z_1$ bzw. $Z_3$ und gegebenenfalls $Z_2$ Wasserstoff ist.

In erster Linie betrifft die Erfindung die in den Beispielen beschriebenen Verbindungen und Salze, vorzugsweise pharmazeutisch annehmbare Salze, wie z.B. Alkalimetallsalze, von entsprechenden salzbildenden Verbindungen, und ganz besonders das 3-Methyl-2-{[5-methyl-3-(2-methylallyl)-4-oxo-thiazolidinyliden]-hydrazono}-4-oxo-5-thiazolidinyl-hydrogensulfat und das Methyl-3-methyl-2-{[5-methyl-3-(2-methylallyl)-4-oxo-thiazolidinyliden]-hydrazono}-4-oxo-2-thiazolidinyl-hydrogenphosphat, vor allem deren Salze, wie pharmazeutisch annehmbaren Salze, wie z.B. die entsprechenden Alkalimetallsalze, wie die Natriumsalze.

Die neuen Verbindungen der allgemeinen Formel I können nach an sich bekannten Verfahren hergestellt werden. So kann man sie herstellen, indem man

a) eine Verbindung der Formel

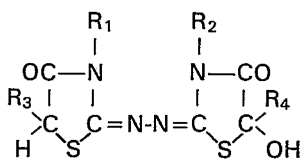

(II)

mit einer, den Rest der Teilformel Ia bzw. Ib einführenden Verbindung, wie im folgenden definiert, umsetzt, oder

b) in einer Verbindung der Formel

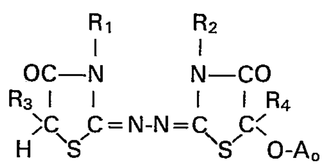

(III)

in welcher $A_o$ einen Rest der Formel

$-SO_2-Y_1$ oder 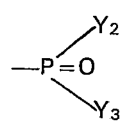

(IIIa) (IIIb)

bedeutet, in welcher $Y_1$ bzw. $Y_3$ einen durch die Gruppe O-$Z_1$ bzw. O-$Z_3$ oder deren Salzform ersetzbaren Rest und $Y_2$ einen Rest O-$Z_2$ oder einen, durch die Gruppe O-$Z_2$ oder deren Salzform ersetzbaren Rest bedeutet, den Rest $Y_1$ bzw. den Rest $Y_3$ und gegebenenfalls den Rest $Y_2$ durch die Gruppe O-$Z_1$ bzw. O-$Z_3$ und gegebenenfalls O-$Z_2$ oder deren Salzform ersetzt, und gewünschtenfalls eine Verbindung der allgemeinen Formel I in eine andere Verbindung der allgemeinen Formel I überführt und/oder gewünschtenfalls ein verfahrensgemäss erhältliches Salz in die freie Verbindung oder in ein anderes Salz überführt, und/oder eine verfahrensgemäss erhältliche Verbindung der Formel I, in welchem $Z_1$ bzw. $Z_3$ und gegebenenfalls $Z_2$ Wasserstoff bedeuten, in ein Salz derselben überführt, und/oder, wenn erwünscht, ein verfahrensgemäss erhältliches Isomerengemisch in die Isomeren auftrennt.

Einen Rest der Formel Ia oder Ib einführende Verbindungen sind Schwefeltrioxid, das auch in Form von Komplexen, wie des Pyridinkomplexes, eingesetzt werden kann, oder Verbindungen der allgemeinen Formeln

$X_1—SO_2—O—Z_1$ bzw. 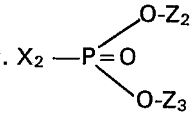

(IV) (V)

in welchen $X_1$ bzw. $X_2$ reaktionsfähiges, funktionell abgewandeltes Hydroxy bedeutet. Letzteres ist. z.B. insbesondere ein mit einer anorganischen oder organischen Säure verestertes Hydroxy, wie mit einer Halogenwasserstoffsäure oder einer Aryl- oder Alkansulfonsäure, z.B. p-Toluolsulfonsäure, Methan- oder Äthansulfonsäure verestertes Hydroxy. Insbesondere ist $X_1$ oder $X_2$ Halogen, wie Brom und vor allem Chlor. Als Ausgangsstoffe der Formel IV kommen z.B. Chlorsulfonsäure und deren Niederalkylester, und als Ausgangsstoffe der Formel V z.B. Diniederalkyl- oder Niederalkylen-phosphorochloridate oder auch entsprechende Phosphorobromidate in Betracht.

Vorzugsweise wird die Umsetzung mit Schwefeltrioxid in einem inerten Lösungsmittel oder Lösungsmittelgemisch, diejenige mit dem Schwefeltrioxid-Pyridinkomplex, z.B. in Methylenchlorid oder Dimethylformamid oder deren Gemischen mit Pyridin, und Umsetzungen mit Schwefeltrioxid z.B. in Dimethylformamid durchgeführt. Die Reaktionstemperaturen liegen zwischen etwa 0° und etwa 100°C; vorzugsweise arbeitet man bei Raumtemperatur oder schwach erhöhten Temperaturen. Bei Verwendung des Schwefeltrioxid-Pyridinkomplexes erhält man als unmittelbares Reaktionsprodukt ein Pyridiniumsalz der Verbindungen der Formel I, das man in die entsprechenden Säuren oder vorzugsweise direkt in andere Salze, wie z.B. Alkalimetallsalze, überführen kann. Bei Verwendung von Schwefeltrioxid entstehen freie Säuren, die gewünschtenfalls direkt, d.h. ohne vorherige Aufarbeitung, in Salze, z.B. Alkalimetallsalze, übergeführt werden können.

Die Umsetzung von Verbindungen der Formel II mit solchen der Formeln IV oder V wird vorzugsweise in einem inerten, insbesondere aprotischen organischen Lösungsmittel, wie z.B. Methylenchlorid, Acetonitril, Dimethylformamid oder Dimethylsulfoxid, und vorzugsweise in Gegenwart eines säurebindenden Mittels, wie einer organischen Base, z.B. Triniederalkylamin, wie Äthyldiisopropylamin oder Tri-äthylamin, ferner z.B. Pyridin oder Imidazol, oder Alkalimetallniederalkoxid, z.B. Natrium-methoxid oder -äthoxid, oder einer anorganischen Base, z.B. Na-trium- oder Kaliumhydroxid, sowie eines basischen Ionenaustauschers durchgeführt. Als Reaktionstemperatur wird z.B. eine solche zwischen 0° und etwa 100°C, vorzugsweise Raumtemperatur oder schwach erhöhte Temperatur gewählt, und die Reaktion kann, wenn notwendig, in einem geschlossenen Gefäss, und/oder in einer Inertgas-, wie Stickstoffatmosphäre, durchgeführt werden.

Die Ausgangsstoffe der Formel II sind bekannt (z.B. deutsche Offenlegungsschrift 2 405 395) oder

sind analog zu den dort beschriebenen Verbindungen herstellbar.

In den Ausgangsstoffen der Formel III sind Reste $Y_1$ bzw. $Y_3$ und gegebenenfalls $Y_2$ z.B. veresterte, wie durch starke Säuren, z.B. durch Mineralsäuren, ferner durch starke organische Säuren veresterte, oder verätherte, z.B. durch aliphatische, cycloaliphatische, aromatische oder araliphatische Reste, wie entsprechende, gegebenenfalls substituierte Kohlenwasserstoffreste verätherte Hydroxygruppen. Veresterte Hydroxygruppen sind insbesondere Halogen, wie Chlor oder Brom, während verätherte Hydroxygruppen u.a. Aryloxy, wie Phenoxy oder p-Nitrophenoxy, oder Arylniederalkoxy, wie insbesondere Benzyloxy, ferner p-Nitrobenzyloxy, sowie Niederalkenyloxy, z.B. Allyloxy, ferner Niederalkoxy, wie z.B. die Gruppen $-OZ_1$, $-OZ_2$ bzw. $-OZ_3$, bedeuten.

Durch Hydrolyse, wie durch die Einwirkung von Wasser, gegebenenfalls in Form von Gemischen mit geeigneten organischen Lösungsmitteln, wie Dioxan oder Niederalkanolen, auf Verbindungen der allgemeinen Formel III mit veresterten Hydroxygruppen, wie Halogen, als Reste $Y_1$, bzw. $Y_3$ und gegebenenfalls $Y_2$, kann man zu Verbindungen der allgemeinen Formel I mit Wasserstoff als $Z_1$ bzw. $Z_3$ und gegebenenfalls $Z_2$ gelangen. Solche Verbindungen können aus Ausgangsstoffen der Formel III, worin $Y_1$, $Y_2$ und/oder $Y_3$ geeignet veresterte Hydroxygruppen darstellen, auch in Abwesenheit von Wasser, z.B. durch Umesterung, wie bei der Einwirkung mit einem geeigneten Alkohol, z.B. α-Methylbenzylalkohol, entstehen. Dieselben Endstoffe kann man durch basische Hydrolyse sowohl aus den vorgenannten Ausgangsstoffen der allgemeinen Formel III, als auch aus solchen mit Aryloxy- oder Aralkoxygruppen als $Y_1$ bzw. $Y_3$ und gegebenenfalls $Y_2$ erhalten, z.B. durch Einwirkung von Basen in Gegenwart von mindestens äquimolaren Mengen Wasser, vorzugsweise in wasserhaltigen organischen Lösungsmitteln, wie entsprechenden Niederalkanolen oder Dioxan. Als Basen kann man hierbei sowohl organische, vorzugsweise tertiäre, wie die weiter oben genannten, oder anorganische Basen, wie Natrium- oder Kaliumhydroxid, verwenden, wobei man die Reaktionsprodukte entweder direkt als Salze oder, nach Behandeln mit einem sauren Reagens, als freie Säuren erhalten kann.

In Ausgangsstoffen der Formel III, in welchen $Y_1$, und insbesondere $Y_3$ und gegebenenfalls $Y_2$ für veräthertes Hydroxy, insbesondere Niederalkoxy und in erster Linie Methoxy stehen, kann ein solcher Rest vorteilhafterweise mittels einer nucleophilen Substitutionsreaktion durch Hydroxy ersetzt werden; dabei kann in einem entsprechenden Ausgangsmaterial, in welchem beide Reste $Y_2$ und $Y_3$ für veräthertes Hydroxy, z.B. Methoxy, stehen, wenn erwünscht, nur eine der verätherten Hydroxygruppen gespalten werden. Die Spaltung kann durch Behandeln des entsprechenden Ausgangsmaterials der Formel III mit einem geeigneten nucleophilen Reagens durchgeführt werden, wobei ein solches vorzugsweise eine verätherbare Hydroxy- oder insbesondere Mercaptogruppe oder eine substituierbare, inkl. quaternisierbare, Aminogruppe enthält. Solche Reagentien sind u.a. eine gegebenenfalls substituierte Thiophenolat-Verbindung, wie Thiophenol in Gegenwart einer anorganischen oder organischen Base, wie Triäthylamin, oder eine geeignete Harnstoff- oder insbesondere Thioharnstoff-Verbindung, wie Thioharnstoff, ferner eine geeignete, vorzugsweise sterisch gehinderte Aminverbindung, wie ein entsprechendes Niederalkylamin, z.B. tert.-Butylamin, ferner Triniederalkylamin, wie Trimethylamin, N-Niederalkyl-morpholin oder -thiomorpholin, z.B. N-Methylmorpholin, oder Pyridin.

Die Spaltung einer verätherten Hydroxygruppe $Y_1$ bzw. $Y_3$ und gegebenenfalls $Y_2$ kann auch mittels Behandeln mit einer starken anorganischen Base, wie einem Alkalimetall-, z.B. Natrium- oder Kaliumhydroxid, vorzugsweise in Gegenwart eines Alkohols, wie Niederalkanols, z.B. Äthanol, oder Ammoniumhydroxid, oder mit einem geeigneten Neutralsalz, insbesondere einem Alkali- oder Erdalkalimetallhalogenid oder -thiocyanat, wie Natriumjodid, Bariumjodid oder Natriumthiocyanat, erreicht werden, wobei sich diese Methode in erster Linie zur Spaltung von Niederalkenyloxy-, z.B. Allyloxy-, oder Arylniederalkoxy-, z.B. Benzyloxygruppen eignet.

Ferner lassen sich geeignet verätherte Hydroxygruppen $Y_1$ bzw. $Y_3$ und gegebenenfalls $Y_2$, insbesondere aromatisch oder araliphatisch verätherte Hydroxygruppen, wie gegebenenfalls substituiertes Phenyloxy der Benzyloxy mittels Hydrogenolyse, wie durch Behandeln mit Wasserstoff in Gegenwart eines Edelmetallkatalysators, wie eines Platin- oder Palladiumkatalysators, spalten, wobei darauf geachtet werden muss, dass eine Niederalkenylgruppe $R_1$ bzw. $R_2$ nicht ebenfalls reduziert wird.

Weiter kann man in Ausgangsstoffen der Formel III veresterte Hydroxygruppen $Y_1$ bzw. $Y_3$ und gegebenenfalls $Y_2$, wie Halogen, durch Niederalkoxy ersetzen, indem man eine entsprechende Verbindung mit einem Niederalkanol in Gegenwart einer Base unter im wesentlichen wasserfreien Reaktionsbedingungen, oder mit einem Niederalkoxid eines Alkali-, Erdalkali- oder Erdmetalls, wie einem Natrium- oder Kaliummethoxid, -äthoxid oder tert.-butoxid, umsetzt.

Die obigen Reaktionen werden in an sich bekannter Weise, in Abwesenheit, vorteilhafterweise jedoch in Anwesenheit eines geeigneten inerten Lösungsmittels, wie eines gegebenenfalls halogenierten Kohlenwasserstoffs, z.B. Benzol oder Methylenchlorid, Niederalkanols, z.B. Methanol, Dimethylsulfoxid oder Acetonitril oder eines Lösungsmittelgemisches, und üblicherweise unter milden Reaktionsbedingungen, vorzugsweise bei Temperaturen zwischen etwa —10°C und etwa 100°C, insbesondere bei Raumtemperatur oder schwach erhöhten Temperaturen bis etwa 50°C, wenn notwendig, in einem geschlossenen Gefäss und/oder unter einer Inertgas-, z.B. Stickstoffatmosphäre, durchgeführt. Dabei können die Reaktionsprodukte in Form der freien Säuren abgetrennt oder direkt in die Salze, z.B. Alkalimetallsalze, übergeführt werden.

Ausgangsstoffe der Formel III, in denen $Y_1$ bzw. $Y_3$ und gegebenenfalls $Y_2$ eine verätherte Hydroxygruppe, wie Niederalkoxy, Aryloxy oder Arylniederalkoxy bedeutet, können gemäss Verfahren a) hergestellt werden.

Ebenfalls analog Verfahren a) kann man zu Ausgangsstoffen der Formel III gelangen, in denen $Y_1$ bzw. $Y_3$ und gegebenenfalls $Y_2$ verestertes Hydroxy, insbesondere Halogen, wie Chlor bedeuten, indem man eine Verbindung der Formel II unter milden Reaktionsbedingungen, z.B. mit der äquimolaren Menge Sulfurylchlorid oder Phosphoroxychlorid umsetzt. Vorzugsweise werden die so erhältlichen Verbindungen der Formel III direkt gemäss Verfahren b) weiter umgesetzt, z.B. durch Behandeln mit Wasser oder einem wasserhaltigen organischen Lösungsmittel, zu Verbindungen der Formel I, in denen $Z_1$ bzw. $Z_3$ und $Z_2$ Wasserstoff bedeuten, oder Salzen davon, oder z.B. durch Behandeln mit Alkalimetallniederalkoxiden, wie Natrium-methoxid oder -äthoxid, zu Verbindungen der Formel I mit Niederalkyl als $Z_1$ bzw. $Z_3$ und gegebenenfalls $Z_2$.

Erfindungsgemäss erhältliche Verbindungen der Formel I können in an sich bekannter Weise in andere Verbindungen der Formel I übergeführt werden. So kann man nach der vorstehenden Verfahrensmodifikation b) Verbindungen der Formel I, worin A die Teilformel Ia oder insbesondere Ib hat, und $Z_1$ bzw. $Z_2$ für Niederalkyl und $Z_3$ für Wasserstoff oder Niederalkyl stehen, wobei eine Niederalkylgruppe $Z_1$ bzw. $Z_2$ in erster Linie Methyl bedeutet, in Verbindungen der Formel I mit der Teilformel Ia bzw. Ib für den Rest A umwandeln, in denen $Z_1$ bzw. $Z_2$ für Wasserstoff und $Z_3$ für Wasserstoff oder Niederalkyl stehen.

Ferner kann man in Verbindungen der Formel I mit den Teilformeln Ia und Ib, worin $Z_1$, $Z_2$ und/oder $Z_3$ für Wasserstoff stehen, diesen, z.B. durch Behandeln der entsprechenden Verbindung oder eines Salzes davon mit einem reaktionsfähigen Ester eines Niederalkanols und einer starken Säure, wie einem entsprechenden Niederalkylhalogenid, z.B. -chlorid, -bromid oder -jodid, oder einem entsprechenden Aren- oder Niederalkansulfonsäure- z.B. p-Toluolsulfonsäure- oder Methansulfonsäure-niederalkylester, durch Niederalkyl ersetzen.

Erfindungsgemäss erhältliche Salze von salzbildenden Verbindungen der Formel I können in an sich bekannter Weise, z.B. durch Behandeln mit einem sauren Reagens, wie einer Säure, in die freien Verbindungen oder durch Umsalzen in andere Salze übergeführt werden.

Salze von zur Salzbildung geeigneten Verbindungen der Formel I, insbesondere pharmazeutisch annehmbare Salze, wie z.B. die obgenannten, können in an sich bekannter Weise, z.B. durch Behandeln mit einer geeigneten Base, wie einem Alkalimetallhydroxid, Ammoniak oder einem salzbildenden Amin, hergestellt werden.

Gemische von Isomeren können in an sich bekannter Weise in die reinen Isomeren aufgetrennt werden, Racematgemische u.a. mittels physikalischer Auftrennung, z.B. fraktionierter Kristallisation oder Destillation, oder Chromatographie, u.a. Hochdruckflüssigkeitschromatographie, und Racemate u.a. unter Bildung von Salzen mit optisch aktiven Basen und Auftrennung der erhaltenen Salzgemische, z.B. durch fraktionierte Kristallisation.

Die Erfindung betrifft auch diejenigen Ausführungsformen des Verfahrens, bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Gegenstand der vorliegenden Erfindung ist ebenfalls die Verwendung der neuen Verbindungen als pharmakologisch aktive, insbesondere als carcinostatisch aktive Verbindungen. Die zur Verwendung gelangenden täglichen Dosen von solchen Verbindungen liegen für Mammalien in Abhängigkeit von der Spezies, dem Alter, dem individuellen Zustand und der Applikationsweise zwischen etwa 2 mg und etwa 250 mg, insbesondere zwischen etwa 5 mg und etwa 100 mg pro Körpergewicht, und innerhalb dieses Bereiches bei parenteraler Applikation, z.B. intramuskulärer oder subcutaner Injektion, oder intravenöser Infusion, im allgemeinen niedriger als bei enteraler, d.h. oraler oder rektaler Applikation. Die Verbindungen der Formel I und pharmazeutisch annehmbaren Salze von solchen mit salzbildenden Eigenschaften werden oral oder rektal vorzugsweise in Doseneinheitsformen, wie Tabletten, Dragées oder Kapseln bzw. Suppositorien, und parenteral insbesondere als injizierbare Lösungen, Emulsionen oder Suspensionen oder als Infusionslösungen verabreicht, wobei als Lösungen in erster Linie solche von Salzen in Betracht kommen.

Ebenfalls Gegenstand der Erfindung sind die pharmazeutischen Präparate zur enteralen, z.B. oralen oder rektalen, oder zur parenteralen Verabreichung, welche eine therapeutisch wirksame Menge einer Verbindung der Formel I oder eines pharmazeutisch annehmbaren Salzes einer solchen mit salzbildenden Eigenschaften, gegebenenfalls zusammen mit einem pharmazeutisch verwendbaren Trägerstoff oder Trägerstoffgemisch enthalten, wobei Trägerstoffe anorganisch oder organisch, fest oder flüssig sein können. Entsprechende Dosiseinheitsformen, insbesondere zur peroralen Verabreichung, z.B. Dragées, Tabletten oder Kapseln, enthalten vorzugsweise von etwa 50 mg bis etwa 500 mg, insbesondere von etwa 100 mg bis etwa 400 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure,

Talk, Stearinsäure oder Salze davon, wie Magnesium- oder Calciumstearat, und/oder Polyäthylenglykol. Dragée-Kerne können mit geeigneten, gegebenenfalls Magensaft-resistenten Überzügen versehen werden, wobei man u.a. konzentrierte Zuckerlösungen, welche gegebenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellungen von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Öle, z.B. Sesamöl, oder synthetische Fettsäureester, z.B. Äthyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung. Temperaturen werden in Celsiusgraden angegeben.

*Beispiel 1*

Zu einer Lösung von 32,8 g (0,1 Mol) 5-Hydroxy-3-methyl-2-{[5-methyl-3-(2-methylallyl)-4-oxo-2-thiazolidinyliden]-hydrazono}-4-thiazolidinon in 700 ml Methylenchlorid und 200 ml wasserfreiem Pyridin gibt man 56 g (0,35 Mol) Schwefeltrioxid-Pyridinkomplex und rührt das Gemisch 20 Stunden bei 20-25°. Dann gibt man 700 ml Wasser zu, rührt weitere 20 Minuten und trennt die beiden Schichten. Die Methylenchloridlösung trocknet man über Magnesiumsulfat und dampft sie im Wasserstrahlvakuum ein. Zum Rückstand gibt man 500 ml Diäthyläther, nutscht das ausgefallene gelbe Reaktionsprodukt ab und wäscht es 3mal mit Aceton und dann mit Diäthyläther nach. Das erhaltene Pyridinium-[3-methyl-2-{[5-methyl-3-(2-methylallyl)-4-oxo-2-thiazolidinyliden]-hydrazono}-4-oxo-5-thiazolidinyl]-sulfat schmilzt bei 187°.

Zur Überführung in das Natriumsalz löst man 48,7 g (0,10 Mol) des obigen Pyridiniumsalzes in 1100 ml Methylenchlorid und 100 ml Methanol und tropft unter gutem Rühren eine, aus 2,3 g (0,10 Mol) Natrium und 30 ml Methanol bereitete Natriummethoxidlösung zu, wobei das gewünschte Natriumsalz ausfällt. Nach Zugabe von 300 ml Äther nutscht man es ab und wäscht es zweimal mit Methylenchlorid, einmal mit Diäthyläther-Methanol 4 : 1 und dann mit Äther nach. Nach dem Trocknen im Hochvakuum bei 60° schmilzt das erhaltene Natrium-[3-methyl-2-{[5-methyl-3-(2-methylallyl)-4-oxo-2-thiazolidinyliden]-hydrazono}-4-oxo-5-thiazolidinyl]-sulfat bei 195° (unter Zersetzung).

*Beispiel 2*

Analog Beispiel 1 erhält man das Pyridinium-{2-{[3-methyl-4-oxo-2-thiazolidinyliden)-hydrazono]-3-(2-methylallyl7-4-oxo-5-thiazolidinyl}-sulfat vom Smp. 161-168°, ausgehend von 31,4 g (0,10 Mol) 5-Hydroxy-2-[(3-methyl-4-oxo-2-thiazolidinylen)-hydrazono]-3-(2-methylallyl)-4-thiazolidinon und 56 g (0,35 Mol) Schwefeltrioxid-Pyridinkomplex; und ebenfalls analog Beispiel 1 das entsprechende Natriumsalz vom Smp. 216° (unter Zersetzung) aus 47,4 g (0,10 Mol) des Pyridiniumsalzes in 800 ml Methylenchlorid und einer Natriummethoxidlösung aus 2,3 g (0,10 Mol) Natrium und 200 ml Methanol.

*Beispiel 3*

Zu einer Lösung von 31,4 g (0,1 Mol) 5-Hydroxy-2-[(3-methyl-4-oxo-thiazolidinyliden)-hydrazono]-3-(2-methylallyl)-4-thiazolidinon in 500 ml Methylenchlorid und 100 ml Pyridin gibt man eine Suspension, die zuvor aus einer Lösung von 23,3 g (0,34 Mol) Chlorsulfonsäure in 400 ml Methylenchlorid durch Zutropfen von 180 ml Pyridin bei einer Reaktionstemperatur von —10° bis 0° und unter einer Stickstoffatmosphäre erhalten worden ist. Das erhaltene Reaktionsgemisch wird während 20 Stunden bei 20-25° gerührt. Dann gibt man 700 ml Wasser zu, rührt weitere 20 Minuten und trennt die beiden Schichten. Die Methylenchloridlösung wird

über Magnesiumsulfat getrocknet und anschliessend im Wasserstrahlvakuum eingedampft. Das so erhaltene Pyridinium-{2-[(3-methyl-4-oxo-2-thiazolidinyliden)-hydrazono]-3-(2-methylallyl)-4-oxo-5--thiazolidinyl}-sulfat schmilzt bei 190-191°.

Zur Überführung in das Natriumsalz löst man 47,3 g (0,10 Mol) des obigen Pyridiniumsalzes in 600 ml Methylenchlorid und 400 ml Dimethylformamid und tropft unter gutem Rühren 75 ml einer 2,95%igen Natriummethoxidlösung in Methanol zu. Durch Versetzen mit 1500 ml Diäthyläther wird das Natriumsalz ausgefällt; es wird abgenutscht, einmal mit einem 4 : 1-Gemisch von Diäthyläther und Methanol und dann mit Diäthyläther nachgewaschen. Nach dem Trocknen im Hochvakuum bei 60°C schmilzt das Natrium-2-{[(3-methyl-4-oxo-2-thiazolidinyliden)-hydrazono]-3-(2-methylallyl)-4-oxo-5-thiazolidinyl}-sulfat bei 216° (unter Zersetzung).

*Beispiel 4*

Analog Beispiel 3 erhält man Natrium{3-allyl-2--[(3-methyl-4-oxo-2-thiazolidinyliden)-hydrazono]--4-oxo-5-thiazolidinyl}-sulfat, Smp. 217° (Zers.); ausgehend von 60,1 g (0,29 Mol) 3-Allyl-5-hydroxy--2-[(3-methyl-4-oxo-2-thiazolidinyliden)-hydrazono]-4-thiazolidinon, 46,6 ml (0,70 Mol) Chlorsulfonsäure und 250 ml Pyridin in 700 ml Methylenchlorid, und, zur Überführung ins Natriumsalz, mit 100 ml einer 3,4%igen Natriummethoxidlösung in Methanol.

*Beispiel 5*

Analog Beispiel 1 erhält man Natrium-{3-allyl-2--[(3-allyl-5-methyl-4-oxo-2-thiazolidinyliden)-hydrazono]-4-oxo-5-thiazolidinyl}-sulfat, Smp. 190° (Zersetzung); ausgehend von 68 g (0,20 Mol) 3-Allyl-2--[(3-allyl-5-methyl-4-oxo-2-thiazolidinyliden)-hydrazono]-5-hydroxy-4-thiazolidinon, 81,6 g (0,7 Mol) Chlorsulfonsäure, 300 ml Pyridin in 400 ml Methylenchlorid und, zur Überführung ins Natriumsalz, 50 ml einer 7,6%igen Natriummethoxidlösung in Methanol.

*Beispiel 6*

In eine Lösung von 33 g (0,10 Mol) 5-Hydroxy-3--methyl-2-{[5-methyl-3-(2-methylallyl)-4-oxo-2--thiazolidinyliden]-hydrazono}-4-thiazolidinon und 43 ml Äthyldiisopropylamin in 250 ml Methylenchlorid lässt man unter Rühren 21 ml (0,2 Mol) Dimethylphosphorochloridat zutropfen. Die Reaktion ist anfänglich leicht exotherm, und man hält die Reaktionstemperatur mittels Kühlen bei 25°. Nach erfolgter Zugabe wird das Reaktionsgemisch noch 2 Stunden bei Raumtemperatur gerührt. Hierauf wird es zuerst mit 100 ml eiskalter 2-n. Salzsäure und dann mit zwei Portionen von je 100 ml Wasser ausgeschüttelt. Die Methylenchloridlösung wird über Magnesiumsulfat getrocknet und im Wasserstrahlvakuum eingedampft. Als Rückstand verbleibt das Dimethyl--[3-methyl-2-{[5-methyl-3-(2-methylallyl)-4-oxo-2--thiazolidinyliden]-hydrazono}-4-oxo-5-thiazolidinyl]-phosphat, das nach einmaliger Umkristallisation aus Diäthyläther bei 99-103° schmilzt.

*Beispiel 7*

Zu einer Lösung von 22 g (0,05 Mol) Dimethyl--[3-methyl-2-{[5-methyl-3-(2-methylallyl)-4-oxo-2--thiazolidinyliden]-hydrazono}-4-oxo-5-thiazolidinyl]-phosphat und 26 ml Thiophenol in 70 ml Dioxan lässt man unter Rühren 56 ml Triäthylamin zutropfen, wobei die Reaktionstemperatur bis auf 40° ansteigt. Dann wird das Reaktionsgemisch weitere zwei Stunden bei Raumtemperatur gerührt. Hierauf gibt man 400 ml Diäthyläther hinzu, wobei sich ein schweres Öl abscheidet.

Die Ätherlösung wird abdekantiert und das zurückgebliebene Öl in 200 ml Isopropanol gelöst und unter Rühren mit einer aus 1,15 g (0,05 Mol) Natrium und 30 ml Methanol bereiteten Natriummethoxidlösung versetzt. Dabei scheidet sich das Natrium-methyl--[3-methyl-2-{[5-methyl-3-(2-methylallyl)-4-oxo-2--thiazolidinyliden]-hydrazono}-4-oxo-5-thiazolidinyl]-phosphat ab. Dieses wird abgenutscht, mit wenig Isopropanol und Diäthyläther nachgewaschen und dann 15 Stunden im Hochvakuum bei 60° getrocknet; Smp. 146-150°.

Bei diesem Produkt handelt es sich um das Diastereomerengemisch, das in die beiden Racemate aufgetrennt werden kann, z.B. mittels Hochdruckflüssigkeitschromatographie mit einer stationären Phase aus Kieselgel mit einer chemisch gebundenen C 18-Phase (z.B. Hibar LiChroCart HPLC Kartusche, gefüllt mit LiChrosorb RP 18, Säulenmasse: 250 x 4 mm, der Firma Merck AG, Darmstadt, BRD) und einer flüssigen Phase, z.B. einem 40 : 40 : 20-Gemisch von Methanol/Wasser/0,01 molares wässriges Natriumdihydrogenphosphat.

*Beispiel 8*

Zu einer Suspension von 30 g (0,10 Mol) 2-[(3--Allyl-4-oxo-2-thiazolidinyliden)-hydrazono]-5-hydroxy-3-methyl-4-thiazolidinon und 21,7 g (0,15 Mol) Dimethylphosphorochloridat in 250 ml Methylenchlorid lässt man unter Rühren bei 5-10° 16,7 ml (0,12 Mol) Triäthylamin zutropfen. Die Reaktion ist leicht exotherm, und die suspendierten Stoffe gehen mit Ausnahme des entstandenen Triäthylamin-hydrochlorids in Lösung. Nach erfolgter Zugabe wird das Reaktionsgemisch noch eine Stunde bei Raumtemperatur gerührt. Hierauf wird es zuerst mit 200 ml eiskaltem Wasser und dann mit 100 ml eiskalter, gesättigter Natriumhydrogencarbonatlösung ausgeschüttelt. Die Methylenchloridlösung wird über Magnesiumsulfat getrocknet und unter vermindertem Druck bis zur beginnenden Kristallisation eingeengt. Man versetzt den Rückstand mit 100 ml Diäthyläther und nutscht das {2-[(3-Allyl-4-oxo-2-thiazolidinyliden)-hydrazono]-3-methyl-4-oxo-5-thiazolidinyl}-dimethylphosphat ab; Smp. 147-148°.

Der Ausgangsstoff kann wie folgt hergestellt werden:

a) 17,1 g (0,10 Mol) 3-Allyl-2,4-thiazolidindion--2-hydrazon [farbloses Öl, vgl. US-PS 3 699 116, Beispiel 8a) bis d)] und 8,0 g (0,11 Mol) Methylisothiocyanat werden in 70 ml Isopropanol unter Rühren 2 Stunden unter Rückfluss gekocht, wobei sich das 3-Allyl-2,4-thiazolidindion-2-(4-methyl-3-thiosemicarbazon) als grobkristalliner Niederschlag abscheidet. Man kühlt mit Eis, nutscht ab und wäscht mit einem 1 : 1-Gemisch von Pentan und Diäthyläther nach, Smp. 148-151°.

b) 11,0 g (0,12 Mol) Glyoxylsäure-monohydrat werden in 40 ml Dioxan gelöst und anschliessend mit 200 ml Tetrachlorkohlenstoff verdünnt. Dann werden unter Rühren 24,4 g (0,10 Mol) 3-Allyl-2,4-thiazolidindion-2-(4-methyl-3-thiosemicarbazon) eingetragen. Hierauf erwärmt man und destilliert, unter gleichzeitigem Zutropfen von 120 ml Tetrachlorkohlenstoff, am absteigenden Kühler 120 ml eines azeotropen Gemisches von Tetrachlorkohlenstoff und Wasser ab. Man kühlt auf 20°, verdünnt den Kristallbrei mit 100 ml Diäthyläther, nutscht die Kristalle ab und wäscht sie mit Diäthyläther nach. Das so erhaltene 2-[(3-Allyl-4-oxo-2-thiazolidinyliden)-hydrazono]-5-hydroxy-3-methyl-4-thiazolidinon schmilzt bei 209-210°.

*Beispiel 9*

Analog Beispiel 8 erhält man das {2-[(3-Allyl-5-methyl-4-oxo-2-thiazolidinyliden)-hydrazono]-3-methyl-4-oxo-5-thiazolidinyl}-dimethylphosphat, Smp. 102-107°; ausgehend von 31,4 g (0,10 Mol) 2-[(3-Allyl-5-methyl-4-oxo-2-thiazolidinyliden)-hydrazono]-5-hydroxy-3-methyl-4-thiazolidinon, 21,7 g (0,15 Mol) Dimethylphosphorochloridat und 16,7 ml (0,12 Mol) Triäthylamin in 250 ml Methylenchlorid.

Der Ausgangsstoff wird wie folgt hergestellt:

a) 11,0 g (0,12 Mol) Glyoxylsäure-monohydrat werden in 40 ml Dioxan gelöst und anschliessend mit 200 ml Tetrachlorkohlenstoff verdünnt. Dann werden unter Rühren 25,8 g (0,10 Mol) 3-Allyl-5-methyl-2,4-thiazolidindion-2-(4-methyl-3-thiosemicarbazon) [vgl. US-PS 3 699 116, Beispiel 8a) bis e)] eingetragen. Hierauf erwärmt man das Reaktionsgemisch und destilliert, unter gleichzeitigem Zutropfen von 120 ml Tetrachlorkohlenstoff, am absteigenden Kühler 120 ml eines azeotropen Gemisches von Tetrachlorkohlenstoff und Wasser ab. Man kühlt auf 20°, verdünnt den Kristallbrei mit 100 ml Diäthyläther und 200 ml Pentan, nutscht die Kristalle ab und wäscht sie mit einem 2 : 1-Gemisch von Pentan und Diäthyläther nach. Das so erhaltene 2-[(3-Allyl-5-methyl-4-oxo-2-thiazolidinyliden)-hydrazono]-5-hydroxy-3-methyl-4-thiazolidinon schmilzt bei 164 bis 166°.

*Beispiel 10*

Zu einer Suspension von 40,8 g (0,10 Mol) {2-[(3-Allyl-4-oxo-2-thiazolidinyliden)-hydrazono]-3-methyl-4-oxo-5-thiazolidinyl}-dimethylphosphat und 20,5 ml (0,20 Mol) Thiophenol in 250 ml Isopropanol lässt man unter Rühren 41,4 ml (0,30 Mol) Triäthylamin zutropfen, wobei die Reaktionstemperatur bis auf 30° ansteigt. Dann wird die klare gelbe Reaktionslösung weitere 4 Stunden bei 35° gerührt. Hierauf wird bei 30-35° eine aus 2,3 g (0,10 Mol) Natrium und 50 ml Methanol zubereitete Natriummethoxidlösung zugetropft. Dabei scheidet sich das Natrium-{2-[(3-allyl-4-oxo-2-thiazolidinyliden)-hydrazono]-3-methyl-4-oxo-5-thiazolidinyl}-methylphosphat ab. Dieses wird abgenutscht und mit Isopropanol und Diäthyläther nachgewaschen. Nach Umkristallisation aus einem 4 : 1-Gemisch von Isopropanol und Wasser schmilzt das Produkt bei 200 bis 205° (unter Zersetzung).

*Beispiel 11*

Analog Beispiel 10 erhält man das Natrium-{2-[(3-allyl-5-methyl-4-oxo-2-thiazolidinyliden)-hydrazono]-3-methyl-4-oxo-5-thiazolidinyl}-methylphosphat, Smp. 190° (Zers.); ausgehend von 42,2 g (0,10 Mol) {2-[(3-Allyl-5-methyl-4-oxo-2-thiazolidinyliden)-hydrazono]-3-methyl-4-oxo-5-thiazolidinyl}-dimethylphosphat, 20,5 ml (0,20 Mol) Thiophenol und 41,4 ml (0,30 Mol) Triäthylamin in 250 ml Isopropanol und, zur Überführung ins Natriumsalz, Behandeln des Produkts mit 23 ml einer 10%igen (G/V) methanolischen Natriummethoxidlösung.

*Beispiel 12*

Zu einem Gemisch von 2,18 g Dimethyl-[3-methyl-2-{[5-methyl-3-(2-methylallyl)-4-oxo-2-thiazolidinyliden]-hydrazono}-4-oxo-5-thiazolidinyl]-phosphat und 12 ml Methylenchlorid wird unter einer Stickstoffatmosphäre innerhalb von 7 Minuten unter Rühren und Kühlen bei 4° eine Lösung von 0,4 g tert.-Butylamin in 5 ml Methylenchlorid zugetropft. Man lässt die Temperatur der klaren gelben Lösung auf Raumtemperatur ansteigen, rührt während 3-1/2 Stunden und gibt 1 ml tert.-Butylamin zu. Man rührt während 16 Stunden bei Raumtemperatur, versetzt das Reaktionsgemisch nochmals mit 2 ml tert.-Butylamin und rührt nochmals während 29-1/2 Stunden. Man verdünnt mit 20 ml Diäthyläther, filtriert den Niederschlag ab und wäscht mit einem 1 : 3-Gemisch von Methylenchlorid und Diäthyläther, dann mit Diäthyläther und erhält so das (N-Methyl-tert.-butylammonium)-methyl-[3-methyl-2-{[5-methyl-3-(2-methylallyl)-4-oxo-2-thiazolidinyliden]-hydrazono}-4-oxo-5-thiazolidinyl]-phosphat, das bei 216-217° (mit Zersetzen) schmilzt und bei Raumtemperatur und unter Hochvakuum während 15 Stunden getrocknet wird. Es kann, z.B. durch Behandeln mit einer methanolischen Natriummethoxidlösung in das Natriumsalz übergeführt werden.

*Beispiel 13*

Ein Gemisch von 2,2 g Dimethyl-[3-methyl-2-{[5-methyl-3-(2-methylallyl)-4-oxo-2-thiazolidinyliden]-hydrazono}-4-oxo-5-thiazolidinyl]-phosphat und 0,38 g Thioharnstoff in 2,5 ml Methanol wird während 6 Stunden unter Rühren bei einer Badtemperatur von 70-80° unter Rückfluss gekocht, wobei eine Lösung entsteht, die man während 16 Stunden stehen lässt, und die dann zu einem Kristallbrei erstarrt. Man verdünnt mit 4 bis 5 ml Diäthyläther, zerkleinert das feste Material, filtriert ab und wäscht mit einem 2 : 1-Gemisch von Diäthyläther und Methanol, dann mit Diäthyläther nach. Das so erhältliche (S-Methylisothiuronium)-methyl-[3-methyl-2-{[3-methyl-3-(2-methylallyl)-4-oxo-2-thiazolidinyliden]-hydrazono}-4-oxo-5-thiazolidinyl]-phosphat schmilzt bei 189-191° (mit Zersetzen) und kann z.B. durch Behandeln mit einer methanolischen Natriummethoxid-Lösung, in das Natriumsalz übergeführt werden.

*Beispiel 14*

Eine Lösung von 49,4 g Natrium-methyl-[3-methyl-2-{[5-methyl-3-(2-methylallyl)-4-oxo-2-thiazolidinyliden]-hydrazono}-4-oxo-5-thiazolidinyl}-phosphat in 800 ml Wasser (deionisiert) wird unter Rühren mit 120 ml 1-n Salzsäure versetzt. Es entsteht eine dicke, halbgallertige Masse, die in 1500 ml Dioxan bei 30-35° gelöst wird. Man verdünnt mit 2500 ml Methylenchlorid, schüttelt das Gemisch und trennt die Schichten. Die wässrige Phase wird abgetrennt und zweimal mit je 200 ml Methylenchlorid extrahiert. Die vereinigten organischen Lösungen werden einmal mit 400 ml eines 1 : 1-Gemisches einer konzentrierten wässrigen Natriumchloridlösung und mit Wasser gewaschen und während 5 Minuten über 200 g Magnesiumsulfat getrocknet. Man filtriert, wäscht mit einem 1 : 2-Gemisch von Dioxan und Methylenchlorid nach, dampft das Filtrat unter vermidertem Druck bei einer Badtemperatur von 45 bis 50° auf ein Volumen von 800 ml ein, wobei ein kristalliner Niederschlag gebildet wird, der abfiltriert und zweimal mit einer kleinen Menge Dioxan und dann mit Diäthyläther gewaschen wird. Man erhält so das Methyl-[3-methyl-2-{[5-methyl-3-(2-methylallyl)-4-oxo-2-thiazolidinyliden]-hydrazono}-4-oxo--5-thiazolidinyl]-hydrogenphosphat, das bei 193 bis 194° schmilzt.

*Beispiel 15*

Eine Suspension von 7 g Methyl-[3-methyl-2--{[5-methyl-3-(2-methylallyl)-4-oxo-2-thiazolidinyliden]-hydrazono}-4-oxo-5-thiazolidinyl]-hydrogenphosphat (Beispiel 14) in 40 ml destilliertem Wasser wird unter Rühren durch Zugabe von etwa 4%igem wässrigem Kaliumhydroxid auf pH 7 gestellt und die schwach-trübe Lösung mit etwa 0,5 g Aktivkohle behandelt und filtriert. Das Filtrat wird unter vermindertem Druck auf ein Gewicht von etwa 15 g eingeengt, und der sirupartige, z.T. feste Substanz enthaltende Rückstand in 50 ml Isopropanol gelöst und unter Rühren portionenweise mit Diäthyläther versetzt. Ein schmieriger Niederschlag entsteht; man gibt eine grössere Menge Diäthyläther zu, dekantiert die überstehende Lösung ab und versetzt den Rückstand mit etwa 40 ml Aceton, wobei ein pulverartiger Niederschlag entsteht. Man gibt wiederum Diäthyläther zu, doch lässt sich der Niederschlag nicht filtrieren und wird mit Hilfe von Aceton in ein anderes Gefäss gespült, mit 20 ml Isopropanol versetzt und mit 150 ml Diäthyläther verdünnt, wobei man das nun filtrierbare Kalium-methyl-[3-methyl-2-{[5-methyl-3-(2-methylallyl)-4-oxo-2-thiazolidinyliden]-hydrazono}-4-oxo-5-thiazolidinyl]-phosphat erhält, das unter vermindertem Druck bei 60° während 24 Stunden getrocknet wird; Smp. 167-170° (ab 177° Zersetzen).

*Beispiel 16*

Eine Suspension von 8 g Methyl-[3-methyl-2--{[5-methyl-3-(2-methylallyl)-4-oxo-2-thiazolidinyliden]-hydrazono}-4-oxo-5-thiazolidinyl]-hydrogenphosphat (Beispiel 14) in 50 ml destilliertem Wasser wird unter Rühren mit einer etwa 4%igen wässrigen Ammoniumhydroxidlösung auf pH 7-8 gestellt. Die schwach trübe Lösung wird mit Aktivkohle geklärt und filtriert und das Filtrat unter vermindertem Druck auf ein Gewicht von 20 g eingeengt. Man verdünnt mit 80 ml Isopropanol und versetzt bis zur beginnenden Trübung mit Diäthyläther. Die Kristallisation kann mittels Animpfen eingeleitet werden; das Ammonium-methyl-[3-methyl-2-{[5-methyl-3-(2-methylallyl)-4-oxo-2-thiazolidinyliden]-hydrazono}-4--oxo-5-thiazolidinyl]-phosphat wird abfiltriert und am Hochvakuum bei Raumtemperatur während 20 Stunden ge-trocknet, Smp. 195-197°.

*Beispiel 17*

Eine Suspension von 1 g Methyl-[3-methyl-2--{[5-methyl-3-(2-methylallyl)-4-oxo-2-thiazolidinyliden]-hydrazono}-4-oxo-5-thiazolidinyl]-hydrogenphosphat (Beispiel 14) in 10 ml destilliertem Wasser wird unter Rühren portionenweise mit einer 5%igen wässrigen Lösung von 2-Hydroxyäthylamin bis zu einem pH-Wert von 7-8 versetzt. Die Lösung wird mit 0,3 g Aktivkohle geklärt, filtriert und unter vermindertem Druck auf ein Gewicht von etwa 2 g eingeengt. Der halbfeste Rückstand wird in 7 ml absolutem Äthanol aufgenommen und bis zur einsetzenden Trübung mit Diäthyläther versetzt. Der so erhaltene Niederschlag wird in etwa 20 ml Methanol gelöst, die Lösung mit einer kleinen Menge Aktivkohle versetzt und filtriert, und das nun klare Filtrat auf ein Volumen von etwa 5 ml eingeengt. Das (2-Hydroxyäthylammonium)-methyl-[3-methyl-2-{[5-methyl-3-(2-methylallyl)-4-oxo-2-thiazolidinyliden]-hydrazono}-4--oxo-5-thiazolidinyl]-phosphat wird durch Zugabe von Diäthyläther zur Kristallisation gebracht und dann abfiltriert, Smp. 186-187°.

*Beispiel 18*

Eine Suspension von 1 g Methyl-[3-methyl-2--{[5-methyl-3-(2-methylallyl)-4-oxo-2-thiazolidinyliden]-hydrazono}-4-oxo-5-thiazolidinyl]-hydrogenphosphat (Beispiel 14) in 10 ml destilliertem Wasser wird unter Rühren portionenweise mit einer 5%igen wässrigen Lösung von Tri-(2-hydroxyäthyl)-amin auf pH 7-8 gestellt. Man erhält eine klare Lösung, die nach etwa 5 Minuten trübe wird und unter vermindertem Druck auf ein Gewicht von 3 g eingeengt wird. Man verdünnt mit 20 ml absolutem Äthanol, rührt mit 0,5 g Aktivkohle und filtriert. Das nun klare Filtrat wird unter vermindertem Druck auf ein Gewicht von etwa 3 g eingeengt, wobei das [Tri-(2--hydroxy-äthyl)-ammonium]-methyl-[3-methyl-2--{[5-methyl-3-(2-methylallyl)-4-oxo-2-thiazolidinyliden]-hydrazono}-4-oxo-5-thiazolidinyl]-phosphat zu kristallisieren beginnt. Man gibt 10 ml absoluten Äthanol und portionenweise 10 ml Diäthyläther zu und filtriert das Salz ab und trocknet unter Hochvakuum während 6 Stunden bei 40°, Smp. 145-146°.

*Beispiel 19*

Lacktabletten, enthaltend 300 mg Natrium-[3--methyl-2-{[5-methyl-3-(2-methylallyl)-4-oxo-2--thiazolidinyliden]-hydrazono}-4-oxo-5-thiazolidinyl]-sulfat, können wie folgt hergestellt werden:

*Zusammensetzung* für 10 000 Tabletten

| | |
|---|---|
| Natrium-[3-methyl-2-{[5-methyl-3-(2--methylallyl)-4-oxo-2-thiazolidinyliden]--hydrazono}-4-oxo-5-thiazolidinyl]--sulfat | 3'000,0 g |
| Maisstärke | 680,0 g |

| | |
|---|---|
| Kolloidale Kieselsäure | 200,0 g |
| Magnesiumstearat | 20,0 g |
| Stearinsäure | 50,0 g |
| Natriumcarboxymethylstärke | 250,0 g |
| Wasser | q.s. |

Ein Gemisch des Natrium-[3-methyl-2-{[5-methyl--3-(2-methylallyl)-4-oxo-2-thiazolidinyliden]-hydrazono}-4-oxo-5-thiazolidinyl]-sulfats, 50 g Maisstärke und der kolloidalen Kieselsäure wird mit einem Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45° während 30 Minuten im Wirbelschichttrockner getrocknet. Das trockene Granulat wird durch ein Sieb von 1 mm Maschenweite gedrückt, mit einer zum voraus gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

Die Presslinge werden in einem Dragierkessel von 45 cm Durchmesser mit einer Lösung von 20 g Schellack und 40 g Hydroxypropyl-methylcellulose (niedere Viskosität) in 110 g Methanol und 1350 g Methylenchlorid durch gleichmässiges Aufsprühen während 30 Minuten überzogen; dabei wird durch gleichzeitiges Einblasen von Luft von 60° getrocknet.

Anstelle des obengenannten Wirkstoffes kann auch diselbe Menge eines anderen Wirkstoffes der vorangehenden Beispiele, wie das Natrium-{3-allyl--2-[(3-methyl-4-oxo-2-thiazolidinyliden)-hydrazono]-4-oxo-5-thiazolidinyl}-sulfat, das Dimethyl-[3--methyl-2-{[5-methyl-3-(2-methylallyl)-4-oxo-2--thiazolidinyliden]-hydrazono}-4-oxo-5-thiazolidinyl]-phosphat, das Natrium-methyl-[3-methyl-2-{[5--methyl-3-(2-methylallyl)-4-oxo-2-thiazolidinyliden]-hydrazono}-4-oxo-5-thiazolidinyl]-phosphat oder das (2-Hydroxyäthylammonium)-methyl-[3--methyl-2-{[5-methyl-3-(2-methylallyl)-4-oxo-2--thiazolidinyliden]-hydrazono}-4-oxo-5-thiazolidinyl]-phosphat, verwendet werden.

*Beispiel 20*

Je 300 mg Natrium-[3-methyl-2-{[5-methyl-3-(2--methylallyl)-4-oxo-2-thiazolidinyliden]-hydrazono}--4-oxo-5-thiazolidinyl]-sulfat vermischt mit 60 g Reisstärke, werden in Hartgelatine-Kapseln abgefüllt.

Anstelle des obigen Wirkstoffes kann auch dieselbe Menge Natrium- oder (2-Hydroxyäthylammonium)-methy-[3-methyl-2-{[5-methyl-3-(2-methylallyl)-4-oxo-2-thiazolidinyliden]-hydrazono}-4-oxo--5-thiazolidinyl]-phosphat verwendet werden.

*Beispiel 21*

Je 5 ml einer sterilen, 4%igen wässrigen Lösung von Natrium-[3-methyl-2-{[5-methyl-3-(2-methylallyl)-4-oxo-2-thiazolidinyliden]-hydrazono}-4-oxo--5-thiazolidinyl]-sulfat, entsprechend 200 mg Wirkstoff, werden in Ampullen eingefüllt und diese verschlossen und geprüft.

Anstelle des obigen Wirkstoffes kann auch dieselbe Menge Natrium- oder (2-Hydroxyäthylammonium)-methyl-[3-methyl-2-{[5-methyl-3-(2-methylallyl)-4-oxo-2-thiazolidinyliden]-hydrazono}-4-oxo--5-thiazolidinyl]-phosphat verwendet werden.

**Patentansprüche für die Vertragsstaaten:**
BE, CH, DE, FR, IT, LI, LU, NL, SE

1. Verbindung der allgemeinen Formel

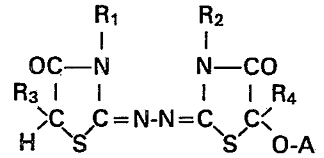

(I)

in welcher eines der Symbole $R_1$ und $R_2$ einen in der 2,3-Stellung ungesättigten Alkylrest mit 3 oder 4 Kohlenstoffatomen und das andere einen solchen Rest oder Niederalkyl mit bis 7 Kohlenstoffatomen bedeuten, $R_3$ und $R_4$ unabhängig voneinander Wasserstoff oder Methyl darstellen, und A einen Rest der Formel

$-SO_2—O—Z_1$ (Ia) oder 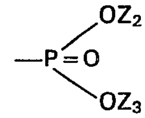 (Ib)

bedeutet, in welchem $Z_1$ bzw. $Z_2$ und $Z_3$ unabhängig voneinander für Wasserstoff oder Niederalkyl mit bis 7 Kohlenstoffatomen stehen, oder $Z_2$ und $Z_3$ zusammen Niederalkylen mit 2 bis 5 Kohlenstoffatomen bedeuten, und Salze von solchen Verbindungen, in denen $Z_1$ bzw. $Z_3$ und gegebenfalls auch $Z_2$ Wasserstoff bedeuten.

2. Verbindung der Formel I gemäss Anspruch 1, in welcher eines der Symbole $R_1$ und $R_2$ Allyl, 1-Methylallyl. 2-Methylallyl oder 2-Propinyl, und das andere ebenfalls eine dieser Gruppen oder Methyl bedeuten, und $R_3$, $R_4$ und A die im Anspruch 1 gegebenen Bedeutungen haben, und Salze von solchen Verbindungen, in denen $Z_1$ bzw. $Z_3$ und gegebenenfalls auch $Z_2$ Wasserstoff bedeuten.

3. Verbindungen der Formel I gemäss Anspruch 1, in welchen einer der Reste $R_1$ und $R_2$ Allyl oder 2-Methylallyl und der andere ebenfalls eine dieser Gruppen oder Methyl bedeuten, $R_3$ und $R_4$ die im Anspruch 1 gegebenen Bedeutungen haben, und A einen Rest der Teilformel Ia mit Wasserstoff als $Z_1$ oder einen Rest der Teilformel Ib mit Niederalkyl mit bis 4 Kohlenstoffatomen als $Z_2$, und Niederalkyl mit bis 4 Kohlenstoffatomen oder Wasserstoff als $Z_3$ bedeutet, und Salze von solchen Verbindungen, in denen $Z_1$ bzw. $Z_3$ und gegebenenfalls auch $Z_2$ Wasserstoff bedeuten.

4. [3-Methyl-2-{[5-methyl-3-(2-methylallyl)-4--oxo-2-thiazolidinyliden]-hydrazono}-4-oxo-5-thiazolidinyl]-hydrogensulfat und seine Salze.

5. {3-Allyl-2-[(3-allyl-5-methyl-4-oxo-2-thiazolidinyliden)-hydrazono]-4-oxo-5-thiazolidinyl}-hydrogensulfat und seine Salze.

6. Dimethyl-[3-methyl-2-{[5-methyl-3-(2-methylallyl)-4-oxo-2-thiazolidinyliden]-hydrazono}-4--oxo-5-thiazolidinyl]-phosphat.

7. Methyl-[3-methyl-2-{[5-methyl-3-(2-methylallyl)-4-oxo-2-thiazolidinyliden]-hydrazono}-4-oxo--5-thiazolidinyl]-hydrogenphosphat und seine Salze.

8. {2-[(3-Allyl-4-oxo-2-thiazolidinyliden)-hydrazono]-3-methyl-4-oxo-5-thiazolidinyl}-methyl-hydrogenphosphat und seine Salze.

9. {2-[(3-Allyl-5-methyl-4-oxo-2-thiazolidinyliden)-hydrazono]-3-methyl-4-oxo-5-thiazolidinyl}-methyl-hydrogenphosphat und seine Salze.

10. Pharmazeutisch verwendbare Salze der Verbindungen gemäss Ansprüchen 1 bis 5 und 7 bis 9.

11. Die Verbindungen der Ansprüche 1 bis 10 zur Verwendung als Tumor-hemmende Wirkstoffe.

12. Die Verbindungen der Ansprüche 1 bis 10 zur Herstellung von pharmazeutischen Präparaten.

13. Pharmazeutische Präparate enthaltend Verbindungen der Ansprüche 1 bis 10.

14. Verfahren zur Herstellung der Verbindungen gemäss Anspruch 1, dadurch gekennzeichnet, dass man

a) eine Verbindung der Formel

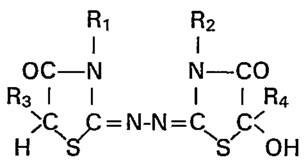

(II)

mit Schwefeltrioxid, gegebenenfalls in Form von Komplexen, oder Verbindungen der allgemeinen Formeln

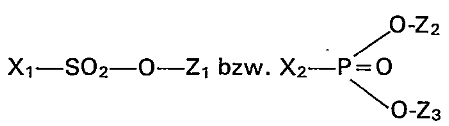

(IV) (V)

in welchen $X_1$ bzw. $X_2$ reaktionsfähiges, funktionell abgewandeltes Hydroxy bedeutet, umsetzt, oder

b) in einer Verbindung der Formel

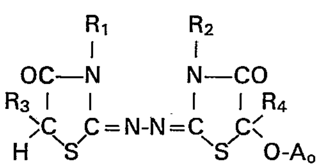

(III)

in welcher $A_o$ einen Rest der Formel

-$SO_2$-$Y_1$ oder 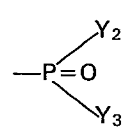

(IIIa) (IIIb)

bedeutet, in welcher $Y_1$ bzw. $Y_3$ einen durch die Gruppe O-$Z_1$ bzw. O-$Z_3$ oder deren Salzform ersetzbaren Rest und $Y_2$ einen Rest O-$Z_2$ oder einen, durch die Gruppe O-$Z_2$ oder deren Salzform ersetzbaren Rest bedeutet, den Rest $Y_1$ bzw. den Rest $Y_3$ und gegebenenfalls den Rest $Y_2$ durch die Gruppe O-$Z_1$ bzw. O-$Z_3$ und gegebenenfalls O-$Z_2$ oder deren Salzform ersetzt, und gewünschtenfalls eine Verbindung der allgemeinen Formel I in eine andere Verbindung der allgemeinen Formel I überführt und/oder gewünschtenfalls ein verfahrensgemäss erhältliches Salz in die freie Verbindung oder in ein anderes Salz überführt, und/oder eine verfahrensgemäss erhältliche Verbindung der Formel I, in welcher $Z_1$ bzw. $Z_3$ und gegebenenfalls $Z_2$ Wasserstoff bedeuten, in ein Salz derselben überführt, und/oder, wenn erwünscht, ein verfahrensgemäss erhältliches Isomerengemisch in die Isomeren auftrennt.

**Patentansprüche für den Vertragsstaat:** AT

1. Verfahren zur Herstellung von Verbindungen der allgemeinen Formel

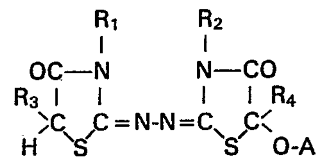

(I)

in welcher eines der Symbole $R_1$ und $R_2$ einen in der 2,3-Stellung ungesättigten Alkylrest mit 3 oder 4 Kohlenstoffatomen und das andere einen solchen Rest oder Niederalkyl mit bis 7 Kohlenstoffatomen bedeuten, $R_3$ und $R_4$ unabhängig voneinander Wasserstoff oder Methyl darstellen, und A einen Rest der Formel

-$SO_2$—O—$Z_1$ oder 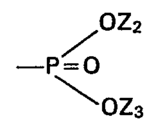

(Ia) (Ib)

bedeutet, in welchem $Z_1$ bzw. $Z_2$ und $Z_3$ unabhängig voneinander für Wasserstoff oder Niederalkyl mit bis 7 Kohlenstoffatomen stehen, oder $Z_2$ und $Z_3$ zusammen Niederalkylen mit 2 bis 5 Kohlenstoffatomen bedeuten, und Salze von solchen Verbindungen, in denen $Z_1$ bzw. $Z_3$ und gegebenenfalls auch $Z_2$ Wasserstoff bedeuten, dadurch gekennzeichnet, dass man

a) eine Verbindung der Formel

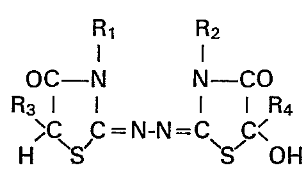

(II)

mit Schwefeltrioxid, gegebenenfalls in Form von Komplexen, oder Verbindungen der allgemeinen Formeln

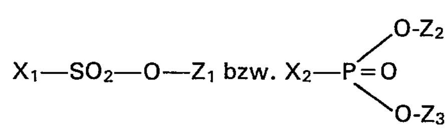

(IV) (V)

in welchen $X_1$ bzw. $X_2$ reaktionsfähiges, funktionell abgewandeltes Hydroxy bedeutet, umsetzt, oder

b) in einer Verbindung der Formel

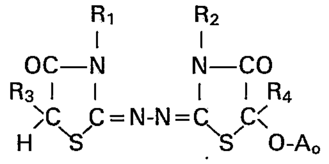

(III)

in welcher $A_o$ einen Rest der Formel

$-SO_2-Y_1$ oder 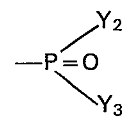

(IIIa) (IIIb)

bedeutet, in welcher $Y_1$ bzw. $Y_3$ einen durch die Gruppe $O-Z_1$ bzw. $O-Z_3$ oder deren Salzform ersetzbaren Rest und $Y_2$ einen Rest $O-Z_2$ oder einen, durch die Gruppe $O-Z_2$ oder deren Salzform ersetzbaren Rest bedeutet, den Rest $Y_1$ bzw. den Rest $Y_3$ und gegebenenfalls den Rest $Y_2$ durch die Gruppe $O-Z_1$ bzw. $O-Z_3$ und gegebenenfalls $O-Z_2$ oder deren Salzform ersetzt, und gewünschtenfalls eine Verbindung der allgemeinen Formel I in eine andere Verbindung der allgemeinen Formel I überführt und/oder gewünschtenfalls ein verfahrensgemäss erhältliches Salz in die freie Verbindung oder in ein anderes Salz überführt, und/oder eine verfahrensgemäss erhältliche Verbindung der Formel I, in welcher $Z_1$ bzw. $Z_3$ und gegebenenfalls $Z_2$ Wasserstoff bedeuten, in ein Salz derselben überführt, und/oder, wenn erwünscht, ein verfahrensgemäss erhältliches Isomerengemisch in die Isomeren auftrennt.

2. Verfahren nach Anspruch 1 zur Herstellung von Verbindungen der Formel I gemäss Anspruch 1, worin eines der Symbole $R_1$ und $R_2$ Allyl, 1-Methylallyl, 2-Methylallyl oder 2-Propinyl, und das andere ebenfalls eine dieser Gruppen oder Methyl bedeuten, und $R_3$, $R_4$ und A die im Anspruch 1 gegebenen Bedeutungen haben, und Salze von solchen Verbindungen, in denen $Z_1$ bzw. $Z_3$ und gegebenenfalls auch $Z_2$ Wasserstoff bedeuten, dadurch gekennzeichnet, dass man (a) eine Verbindung der Formel II gemäss Anspruch 1, worin $R_1$, $R_2$, $R_3$ und $R_4$ die oben gegebenen Bedeutungen haben, mit Schwefeltrioxid oder einer, einen Rest der im Anspruch 1 angegebenen Teilformel Ia bzw. Ib abgebenden Verbindung umsetzt, oder (b) in einer Verbindung der Formel III, worin $R_1$, $R_2$, $R_3$ und $R_4$ die oben gegebenen Bedeutungen haben und $A_o$ einen Rest der Formel IIIa oder IIIb bedeutet, worin $Y_1$, $Y_2$ und $Y_3$ die im Anspruch 1 gegebenen Bedeutungen haben, den Rest $Y_1$ bzw. den Rest $Y_3$ und gegebenenfalls den Rest $Y_2$ durch die Gruppe $O-Z_1$ bzw. $O-Z_3$ und gegebenenfalls $O-Z_2$ oder deren Salzform ersetzt, und, wenn erwünscht, ein Reaktionsprodukt der allgemeinen Formel I, in welchem $Z_1$ bzw. $Z_3$ und gegebenenfalls $Z_2$ Wasserstoff bedeuten, in ein Salz mit einer Base überführt oder aus einem erhaltenen Salz die entsprechende Säure freisetzt und gewünschtenfalls eine Verbindung der allgemeinen Formel I in an sich bekannter Weise in eine andere Verbindung der allgemeinen Formel I oder ein Salz derselben überführt.

3. Verfahren nach Anspruch 1, dadurch gekennzeichnet, dass man Schwefeltrioxid oder einen Komplex davon als eine den Rest der Teilformel Ia einführende Verbindung verwendet.

4. Verfahren nach Anspruch 1, dadurch gekennzeichnet, dass man eine Verbindung der Formel

$X_1-SO_2-O-Z_1$ bzw. 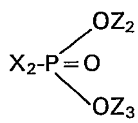

worin $X_1$ bzw. $X_2$ reaktionsfähiges funktionell abgewandeltes Hydroxy darstellen, als eine, den Rest der Formel Ia bzw. Ib einführende Verbindung verwendet.

5. Verfahren nach Anspruch 2, dadurch gekennzeichnet, dass man eine Verbindung der Formel

$X_1-SO_2-O-Z_1$ bzw. 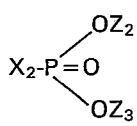

worin $X_1$ bzw. $X_2$ reaktionsfähiges funktionell abgewandeltes Hydroxy darstellen, als eine den Rest der Formel Ia bzw. Ib einführende Verbindung verwendet.

6. Verfahren nach Anspruch 1, dadurch gekennzeichnet, dass $Y_1$ bzw. $Y_2$ und/oder $Y_3$ eine veresterte oder eine verätherte Hydroxygruppe darstellt.

7. Verfahren nach Anspruch 2, dadurch gekennzeichnet, dass $Y_1$ bzw. $Y_2$ und/oder $Y_3$ eine veresterte oder eine verätherte Hydroxygruppe darstellt.

8. Verfahren nach Anspruch 6, dadurch gekennzeichnet, dass man eine verätherte Hydroxygruppe mittels einer nucleophilen Substitutionsreaktion durch Hydroxy ersetzt.

9. Verfahren nach Anspruch 8, dadurch gekennzeichnet, dass man ein geeignetes nucleophiles Reagens verwendet, das eine verätherbare Hydroxy- oder Mercaptogruppe oder eine substituierbare, inkl. quaternisierbare Aminogruppe enthält.

10. Verfahren nach Anspruch 7, dadurch gekennzeichnet, dass man eine verätherte Hydroxygruppe durch Behandeln mit einer Thiophenolat-Verbindung durch Hydroxy ersetzt.

11. Verfahren nach einem der Ansprüche 1, 3, 4, 6, 8 und 9, dadurch gekennzeichnet, dass man Verbindungen der Formel I gemäss Anspruch 1 herstellt, in welchen einer der Reste $R_1$ und $R_2$ Allyl oder 2-Methylallyl und der andere ebenfalls eine dieser Gruppen oder Methyl bedeuten, $R_3$ und $R_4$ die im Anspruch 1 gegebenen Bedeutungen haben, und A einen Rest der Teilformel Ia mit Wasserstoff als $Z_1$ oder einen Rest der Teilformel Ib mit Niederalkyl mit bis 4 Kohlenstoffatomen als $Z_2$, und Niederalkyl mit bis 4 Kohlenstoffatomen oder Wasserstoff als $Z_3$ bedeutet, und Salze von solchen Verbindungen, in denen $Z_1$ bzw. $Z_3$ und gegebenenfalls auch $Z_2$ Wasserstoff bedeuten.

12. Verfahren nach einem der Ansprüche 2, 5, 7 und 10, dadurch gekennzeichnet, dass man Verbindungen der Formel I gemäss Anspruch 1 herstellt, in welchen einer der Reste $R_1$ und $R_2$ Allyl oder 2-Methylallyl und der andere ebenfalls eine dieser

Gruppen oder Methyl bedeuten, $R_3$ und $R_4$ die im Anspruch 1 gegebenen Bedeutungen haben, und A einen Rest der Teilformel Ia mit Wasserstoff als $Z_1$ oder einen Rest der Teilformel Ib mit Niederalkyl mit bis 4 Kohlenstoffatomen als $Z_2$, und Niederalkyl mit bis 4 Kohlenstoffatomen oder Wasserstoff als $Z_3$ bedeutet, und Salze von solchen Verbindungen, in denen $Z_1$ bzw. $Z_3$ und gegebenenfalls auch $Z_2$ Wasserstoff bedeuten.

13. Verfahren nach einem der Ansprüche 1, 3, 4, 6, 8 und 9, dadurch gekennzeichnet, dass man das Methyl-[3-methyl-2-{[5-methyl-3-(2-methylallyl)-4--oxo-2-thiazolidinyliden]-hydrazono}-4-oxo-5-thiazolidinyl]-hydrogenphosphat und seine Salze herstellt.

14. Verfahren nach einem der Ansprüche 2, 5, 7 und 10, dadurch gekennzeichnet, dass man das Methyl-[3-methyl-2-{[5-methyl-3-(2-methylallyl)-4--oxo-2-thiazolidinyliden]-hydrazono}-4-oxo-5-thiazolidinyl]-hydrogenphosphat und seine Salze herstellt.

**Claims for the Contracting States:**
DE, FR, CH, LI, IT, NL, BE, SE, LU

1. A compund of the general formula

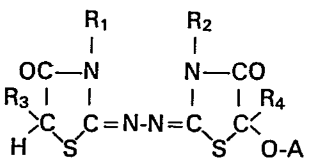

(I)

in which one of the symbols $R_1$ and $R_2$ represents an alkyl radical that has 3 or 4 carbon atoms and is unsaturated in the 2,3-position, and the other represents such a radical or lower alkyl containing up to 7 carbon atoms, $R_3$ and $R_4$ each represents, independently of the other, hydrogen or methyl, and A represents a radical of the formula

$-SO_2-O-Z_1$ or 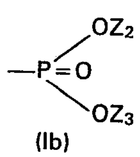

(Ia) (Ib)

in which $Z_1$, or each of $Z_2$ and $Z_3$ independently of the other, represents hydrogen or lower alkyl containing up to 7 carbon atoms, or $Z_2$ and $Z_3$ together represent lower alkylene containing 2 to 5 carbon atoms, or a salt of such a compound in which $Z_1$, or $Z_3$ and optionally also $Z_2$, represent(s) hydrogen.

2. A compound of the formula I according to claim 1 in which one of the symbols $R_1$ and $R_2$ represents allyl, 1-methallyl, 2-methallyl or 2-propynyl, and the other also represents one of these groups or methyl, and $R_3$, $R_4$ and A are as defined in claim 1, or a salt of such a compound, in which $Z_1$, or $Z_3$ and optionally also $Z_2$, represent(s) hydrogen.

3. A compound of the formula I according to claim 1 in which one of the radicals $R_1$ and $R_2$ represents allyl or 2-methallyl, and the other also represents one of these groups or methyl, $R_3$ and $R_4$ are as defined in claim 1, and A represents a radical of the partial formula Ia in which $Z_1$ represents hydrogen, or represents a radical of the partial formula Ib in which $Z_2$ represents lower alkyl containing up to 4 carbon atoms and $Z_3$ represents lower alkyl containing up to 4 carbon atoms or hydrogen, or a salt of such a compound in which $Z_1$, or $Z_3$ and optionally also $Z_2$, represent(s) hydrogen.

4. [3-Methyl-{[5-methyl-3-(2-methallyl)-4-oxo--2-thiazolidinylidene]-hydrazono}-4-oxo-5-thiazolidinyl]-hydrogen sulphate or a salt thereof.

5. {3-Allyl-2-[(3-allyl-5-methyl-4-oxo-2-thiazolidinylidene)-hydrazono]-4-oxo-5-thiazolidinyl}-hydrogen sulphate or a salt thereof.

6. Dimethyl-[3-methyl-2-{[5-methyl-3-(2-methallyl)-4-oxo-2-thiazolidinylidene]-hydrazono}-4-oxo--5-thiazolidinyl]-phosphate.

7. Methyl-[3-methyl-2-{[5-methyl-3-(2-methallyl)-4-oxo-2-thiazolidinylidene]-hydrazono}-4-oxo-5--thiazolidinyl]-hydrogen phosphate or a salt thereof.

8. {2-[(3-Allyl-5-methyl-4-oxo-2-thiazolidinylidene)-hydrazono-3-methyl-4-oxo-5-thiazolidinyl}--methyl phosphate or a salt thereof.

9. {2-[(3-Allyl-4-oxo-2-thiazolidinylidene)-hydrazono]-3-methyl-4-oxo-5-thiazolidinyl}-methyl-hydrogen phosphate or a salt thereof.

10. A pharmaceutically acceptable salt of a compound according to any one of claims 1 to 5 and 7 to 9.

11. Use of a compound according to any one of claims 1 to 10 as tumor inhibitor.

12. Use of a compound according to any one of claims 1 to 10 for the preparation of a pharmaceutical composition.

13. A pharmaceutical composition containing a compound according to any one of claims 1 to 10.

14. A process for the preparation of a compound according to claim 1, which process comprises

a) reacting a compound of the formula

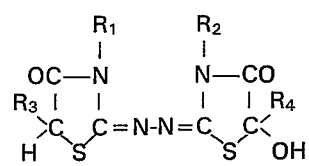

(II)

with sulfur trioxide, which may be in the form of a complex, or with a compound of the general formula

$X_1-SO_2-O-Z_1$ or 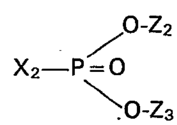

(IV) (V).

in which $X_1$ or $X_2$ represents reactive functionally modified hydroxy, or

b) in a compound of the formula

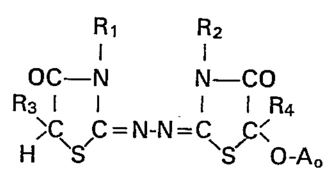

(III)

in which $A_o$ represents a radical of the formula

$-SO_2-Y_1$ or 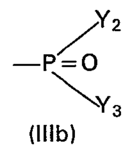

(IIIa) (IIIb)

in which $Y_1$ or $Y_3$ represents a radical that can be replaced by the group $O-Z_1$ or $O-Z_3$, respectively, or by a salt form thereof, and $Y_2$ represents a radical $O-Z_2$ or a radical that can be replaced by the group $O-Z_2$ or by a salt form thereof, replacing the radical $Y_1$ or the radical $Y_3$ by the group $O-Z_1$ or $O-Z_3$, respectively, or by a salt form thereof, and optionally replacing the radical $Y_2$ by the group $O-Z_2$ or by a salt form thereof, and, if desired, converting a compound of the general formula I into a different compound of the general formula I, and/or, if desired, converting a salt obtainable according to the process into the free compound or into a different salt, and/or converting a compound of the formula I obtainable according to the process in which $Z_1$, or $Z_3$ and optionally also $Z_2$, represent(s) hydrogen, into a salt thereof, and/or, if desired, separating an isomeric mixture obtainable according to the process into the isomers.

**Claims for the Contracting State:** AT

1. A process for the preparation of a compound of the general formula

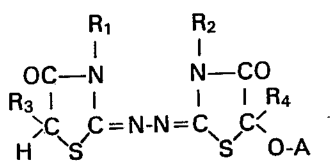

(I)

in which one of the symbols $R_1$ and $R_2$ represents an alkyl radical that has 3 or 4 carbon atoms and is unsaturated in the 2,3-position, and the other represents such a radical or lower alkyl containing up to 7 carbon atoms, $R_3$ and $R_4$ each represents, independently of the other, hydrogen or methyl, and A represents a radical of the formula

$-SO_2—O—Z_1$ or 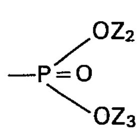

(Ia) (Ib)

in which $Z_1$, or each of $Z_2$ and $Z_3$ independently of the other, represents hydrogen or lower alkyl containing up to 7 carbon atoms, or $Z_2$ and $Z_3$ together represent lower alkylene containing 2 to 5 carbon atoms, or a salt of such a compound in which $Z_1$, or $Z_3$ and optionally also $Z_2$, represent(s) hydrogen, which process comprises

a) reacting a compound of the formula

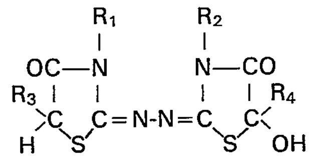

(II)

with sulfur trioxide, which may be in the form of a complex, or with a compound of the general formula

$X_1—SO_2—O—Z_1$ or 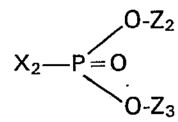

(IV) (V)

in which $X_1$ or $X_2$ represents reactive functionally modified hydroxy, or

b) in a compound of the formula

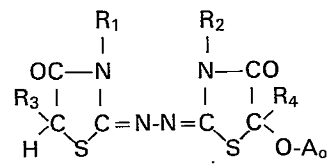

(III)

in which $A_o$ represents a radical of the formula

$-SO_2-Y_1$ or 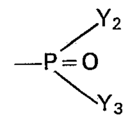

(IIIa) (IIIb)

in which $Y_1$ or $Y_3$ represents a radical that can be replaced by the group $O-Z_1$ or $O-Z_3$, respectively, or by a salt form thereof, and $Y_2$ represents a radical $O-Z_2$ or a radical that can be replaced by the group $O-Z_2$ or by a salt form thereof, replacing the radical $Y_1$ or the radical $Y_3$ by the group $O-Z_1$ or $O-Z_3$, respectively, or by a salt form thereof, and optionally replacing the radical $Y_2$ by the group $O-Z_2$ or by a salt form thereof, and, if desired, converting a compound of the general formula I into a different compound of the general formula I, and/or, if desired, converting a salt obtainable according to the process into the free compound or into a different salt, and/or converting a compound of the formula I obtainable according to the process in which $Z_1$, or $Z_3$ and optionally also $Z_2$, represent(s) hydrogen, into a salt thereof, and/or, if desired, separating an isomeric mixture obtainable according to the process into the isomers.

2. A process according to claim 1 for the preparation of a compound of formula I according to claim 1, in which one of the symbols $R_1$ and $R_2$ represents allyl, 1-methallyl, 2-methallyl or 2-propynyl, and the other also represents one of these groups or methyl, and $R_3$, $R_4$ and A are as defined in claim 1, or a salt of such a compound, in which $Z_1$, or $Z_3$ and optionally also $Z_2$, represent(s) hydrogen, which process comprises (a) reacting a compound of the formula II according to claim 1 in which $R_1$, $R_2$, $R_3$ and $R_4$ are as defined above with sulfur trioxide or with a compound that yields a radical of the partial formula Ia or Ib given in claim 1, or, (b) in a compound of the formula III in which $R_1$, $R_2$, $R_3$ and $R_4$ are as defined above

and $A_0$ represents a radical of the formula IIIa or IIIb in which $Y_1$, $Y_2$ and $Y_3$ are as defined in claim 1, replacing the radical $Y_1$ or the radical $Y_3$ by the group O-$Z_1$ or O-$Z_3$, respectively, or by a salt form thereof, and optionally replacing the radical $Y_2$ is replaced by the group O-$Z_2$ or by a salt form thereof, and, if desired, converting a reaction product of the general formula I in which $Z_1$, or $Z_3$ and optionally also $Z_2$, represent(s) hydrogen, into a salt with a base, or freeing the corresponding acid from a resultant salt, and, if desired, converting a compound of the general formula I in a manner known per se into a different compound of the general formula I or into a salt thereof.

3. A process according to claim 1, in which process sulfur trioxide or a complex thereof is used as a compound that yields a radical of the partial formula Ia.

4. A process according to claim 1, in which process a compound of the formula

$X_1$-$SO_2$-O-$Z_1$ or 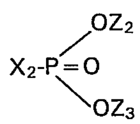

in which $X_1$ or $X_2$ represents reactive functionally modified hydroxy is used as a compound that yields a radical of the formula Ia or Ib.

5. A process according to claim 2, in which process a compound of the formula

$X_1$-$SO_2$-O-$Z_1$ or 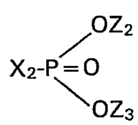

in which $X_1$ or $X_2$ represents reactive functionally modified hydroxy is used as a compound that yields a radical of the formula Ia or Ib.

6. A process according to claim 1, in which $Y_1$, or $Y_2$ and/or $Y_3$, represent(s) an esterified or etherified hydroxyl group.

7. A process according to claim 2, in which $Y_1$, or $Y_2$ and/or $Y_3$, represent(s) an esterified or etherified hydroxyl group.

8. A process according to claim 6, which process comprises replacing an etherified hydroxyl group by hydroxy by means of a nucleophilic substitution reaction.

9. A process according to claim 8, which process comprises using a suitable nucleophilic reagent which contains a hydroxyl or mercapto group capable of being etherified or an amino group capable of being substituted, including quaternised.

10. A process according to claim 7, which process comprises replacing an etherified hydroxyl group by hydroxy by treatment with a thiophenolate compound.

11. A process according to any one of claims 1, 3, 4, 6, 8 and 9, which process comprises preparing a compound of formula I according to claim 1, in which one of the radicals $R_1$ and $R_2$ represents allyl or 2-methallyl, and the other also represents one of these groups or methyl, $R_3$ and $R_4$ are as defined in claim 1, and A represents a radical of the partial formula Ia in which $Z_1$ represents hydrogen, or represents a radical of the partial formula Ib in which $Z_2$ represents lower alkyl containing up to 4 carbon atoms and $Z_3$ represents lower alkyl containing up to 4 carbon atoms or hydrogen, or a salt of such a compound in which $Z_1$, or $Z_3$ and optionally also $Z_2$, represent(s) hydrogen.

12. A process according to any one of claims 2, 5, 7 and 10, which process comprises preparing a compound of formula I according to claim 1, in which one of the radicals $R_1$ and $R_2$ represents allyl or 2-methallyl, and the other also represents one of these groups or methyl, $R_3$ and $R_4$ are as defined in claim 1, and A represents a radical of the partial formula Ia in which $Z_1$ represents hydrogen, or represents a radical of the partial formula Ib in which $Z_2$ represents lower alkyl containing up to 4 carbon atoms and $Z_3$ represents lower alkyl containing up to 4 carbon atoms or hydrogen, or a salt of such a compound in which $Z_1$, or $Z_3$ and optionally also $Z_2$, represent(s) hydrogen.

13. A process according to any one of claims 1, 3, 4, 6, 8 and 9, which process comprises preparing methyl-[3-methyl-2-{[5-methyl-3-(2-methallyl)-4--oxo-2-thiazolidinylidene]-hydrazono}-4-oxo-5-thiazolidinyl]-hydrogen phosphate or a salt thereof.

14. A process according to any one of claims 2, 5, 7 and 10, which process comprises preparing methyl-[3-methyl-2-{[5-methyl-3-(2-methallyl)-4--oxo-2-thiazolidinylidene]-hydrazono}-4-oxo-5-thiazolidinyl]-hydrogen phosphate or a salt thereof.

**Revendications pour les Etats contractants:**
DE, FR, CH, LI, IT, NL, BE, SE, LU

1. Composés de formule générale

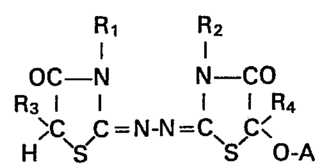

(I)

dans laquelle l'un des symboles $R_1$ et $R_2$ représente un groupe alkyle à 3 ou 4 atomes de carbone insaturé en position 2,3 et l'autre un reste du même type ou un groupe alkyle inférieur contenant jusqu'à 7 atomes de carbone, $R_3$ et $R_4$ représentent chacun, indépendamment l'un de l'autre, l'hydrogène ou un groupe méthyle, et A représente un reste de formule

$-SO_2—O—Z_1$ ou 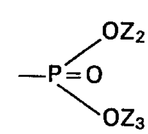

(Ia) (Ib)

dans lequel $Z_1$ d'une part, $Z_2$ et $Z_3$ d'autre part représentent chacun, indépendamment les uns des autres, l'hydrogène ou un groupe alkyle inférieur contenant jusqu'à 7 atomes de carbone, ou bien $Z_2$

et $Z_3$ forment ensemble un groupe alkylène inférieur contenant 2 à 5 atomes de carbone, et les sels de ces composés pour lesquels $Z_1$ et $Z_3$ respectivement et le cas échéant également $Z_2$ représentent l'hydrogène.

2. Composés de formule I selon la revendication 1, dans laquelle l'un des symboles $R_1$ et $R_2$ représente un groupe allyle, 1-méthylallyle, 2-méthylallyle ou 2-propynyle, et l'autre représente également l'un de ces groupes ou un groupe méthyle, et $R_3$, $R_4$ et A ont les significations indiquées dans la revendication 1, et les sels de ces composés pour lesquels $Z_1$ et respectivement $Z_3$ et le cas échéant également $Z_2$ représentent l'hydrogène.

3. Composés de formule I selon la revendication 1, dans laquelle l'un des symboles $R_1$ et $R_2$ représente un groupe allyle ou 2-méthylallyle et l'autre représente également l'un de ces groupes ou un groupe méthyle, $R_3$ et $R_4$ ont les significations indiquées dans la revendication 1, et A représente un reste de formule partielle Ia dans laquelle $Z_1$ représente l'hydrogène ou un reste de formule partielle Ib dans laquelle $Z_2$ représente un groupe alkyle inférieur contenant jusqu'à 4 atomes de carbone, et $Z_3$ représente un groupe alkyle inférieur contenant jusqu'à 4 atomes de carbone ou l'hydrogène, et les sels de ces composés pour lesquels $Z_1$ et $Z_3$ respectivement et les échéant également $Z_2$ représentent l'hydrogène.

4. Le [[3-méthyl-2-{[5-méthyl-3-(2-méthylallyl)-4-oxo-2-thiazolidinylidène]-hydrazono}-4-oxo-5-thiazolidinyl]-hydrogénosulfate et ses sels.

5. Le {3-allyl-2-[(3-allyl-5-méthyl-4-oxo-2-thiazolidinylidène)-hydrazono]-4-oxo-5-thiazolidinyl}-hydrogénosulfate et ses sels.

6. Le [3-méthyl-2-{[5-méthyl-3-(2-méthylallyl)-4-oxo-2-thiazolidinylidène]-hydrazono}-4-oxo-5-thiazolidinyl]-phosphate de diméthyle.

7. Le méthyl-[3-méthyl-2-{[5-méthyl-3-(2-méthylallyl)-4-oxo-2-thiazolidinylidène]-hydrazono}-4-oxo-5-thiazolidinyl]-hydrogénophosphate et ses sels.

8. Le {2-[(3-allyl-4-oxo-2-thiazolidinylidène)-hydrazono]-3-méthyl-4-oxo-5-thiazolidinyl}-méthyl-hydrogénophosphate et ses sels.

9. Le {2-[(3-allyl-5-méthyl-4-oxo-2-thiazolidinylidène)-hydrazono]-3-méthyl-4-oxo-5-thiazolidinyl}-méthyl-hydrogénophosphate et ses sels.

10. Sels acceptables pour l'usage pharmaceutique des composés selon les revendications 1 à 5 et 7 à 9.

11. Les composés des revendications 1 à 10, pour l'utilisation en tant que substances actives inhibant les tumeurs.

12. Les composés des revendications 1 à 10, pour la préparation de compositions pharmaceutiques.

13. Compositions pharmaceutiques contenant des composés des revendications 1 à 10.

14. Procédé de préparation des composés selon la revendication 1, caractérisé en ce que:

a) on fait réagir un composé de formule

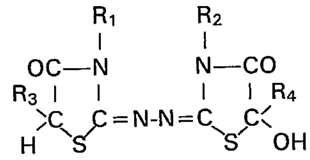
(II)

avec l'anhydride sulfurique, éventuellement à l'état de complexe, ou avec des composés de formules générales respectives

$X_1—SO_2—O—Z_1$ et 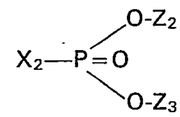

(IV) (V)

dans lesquelles $X_1$ et $X_2$ représentent respectivement des groupes hydroxy soumis à modification fonctionnelle et réactifs, ou bien

b) dans un composé de formule

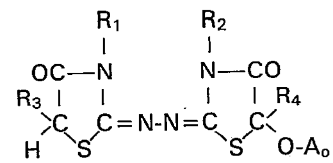
(III)

dans laquelle $A_o$ représente un reste de formule

$-SO_2-Y_1$ ou 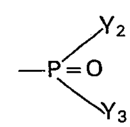

(IIIa) (IIIb)

dans laquelle $Y_1$ et $Y_3$ représentent respectivement un reste remplaçable par le groupe $O-Z_1$ et le groupe $O-Z_3$ respectivement, éventuellement à l'état de sels, $Y_2$ représente un reste $O-Z_2$ ou un reste remplaçable par le groupe $O-Z_2$ éventuellement à l'état de sel, on remplace respectivement le reste $Y_1$ et le reste $Y_3$ et le cas échéant le reste $Y_2$ par le groupe $O-Z_1$ et le groupe $O-Z_3$ respectivement et le cas échéant $O-Z_2$ éventuellement à l'état de sels, et si on le désire, on convertit un composé de formule générale I en un autre composé de formule générale I et/ou, si on le désire, on convertit un sel obtenu conformément à l'invention en le composé libre ou en un autre sel, et/ou on convertit un composé de formule I obtenu conformément à l'invention dans lequel $Z_1$ et $Z_3$ respectivement et le cas échéant $Z_2$ représentent l'hydrogène, en un sel, et/ou, si on le désire, on sépare un mélange d'isomères obtenu conformément à l'invention en les isomères.

**Revendications pour l'Etat Contractant:** AT

1. Procédé de préparation de composés de formule générale

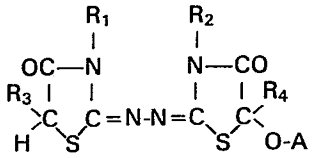

(I)

dans laquelle l'un des symboles $R_1$ et $R_2$ représente un groupe alkyle en C 3 ou C 4 insaturé en position 2,3 et l'autre représente un reste analogue ou un groupe alkyle inférieur contenant jusqu'à 7 atomes de carbone, $R_3$ et $R_4$ représentent chacun, indépendamment l'un de l'autre, l'hydrogène ou un groupe méthyle et A représente un reste de formule

$-SO_2-O-Z_1$ ou 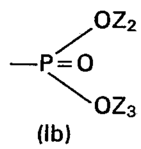

(Ia) (Ib)

dans laquelle $Z_1$ d'une part, $Z_2$ et $Z_3$ d'autre part, représentent chacun, indépendamment les uns des autres, l'hydrogène ou un groupe alkyle inférieur contenant jusqu'à 7 atomes de carbone, ou bien $Z_2$ et $Z_3$ forment ensemble un groupe alkylène inférieur contenant 2 à 5 atomes de carbone, et les sels de ces composés pour lesquels $Z_1$ ou $Z_3$ et le cas échéant également $Z_2$ représentent l'hydrogène, ce procédé se caractérisant en ce que

a) on fait réagir un composé de formule

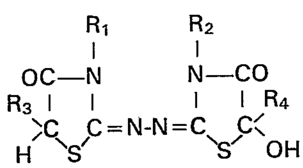

(II)

avec l'anhydride sulfurique, éventuellement à l'état de complexe, ou avec des composés de formules générales respectives

$X_1-SO_2-O-Z_1$ bzw. 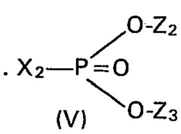

(IV) (V)

dans lesquelles $X_1$ et $X_2$ représentent respectivement des groupes hydroxy à modification fonctionnelle et réactifs, ou bien

b) dans un composé de formule

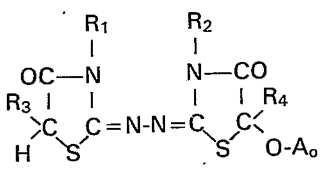

(III)

dans laquelle $A_o$ représente un reste de formule

$-SO_2-Y_1$ ou 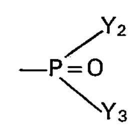

(IIIa) (IIIb)

dans laquelle $Y_1$ et $Y_3$ représentent respectivement un reste remplaçable par le groupe $O-Z_1$ ou $O-Z_3$ respectivement, éventuellement à l'état de sels, et $Y_2$ représente un reste $O-Z_2$ ou un reste remplaçable par le groupe $O-Z_2$ éventuellement à l'état de sel, on remplace respectivement le reste $Y_1$ ou le reste $Y_3$ et le cas échéant le reste $Y_2$ par le groupe $O-Z_1$ ou $O-Z_3$ respectivement et le cas échéant $O-Z_2$, éventuellement à l'état de sel, et si on le désire, on convertit un composé de formule générale I en un autre composé de formule générale I et/ou, si on le désire, on convertit un sel obtenu conformément à l'invention en le composé libre ou en un autre sel, et/ou on convertit un composé de formule I obtenu conformément à l'invention dans lequel $Z_1$ ou $Z_3$ et le cas échéant $Z_2$ représentent l'hydrogène, en un sel, et/ou, si on le désire, on sépare un mélange d'isomères obtenu conformément à l'invention en les isomères.

2. Procédé selon la revendication 1 pour la préparation des composés de formule I selon la revendication 1, dans laquelle l'un des symboles $R_1$ et $R_2$ représente un groupe allyle, 1-méthylallyle, 2-méthylallyle ou 2-propynyle, et l'autre représente aussi l'un de ces groupes ou bien un groupe méthyle, et $R_3$, $R_4$ et A ont les signification indiquées dans la revendication 1, et de sels de ces composés pour lesquels $Z_1$ ou $Z_3$ et le cas échéant également $Z_2$ représentent l'hydrogène, caractérisé en ce que: a) on fait réagir un composé de formule II selon la revendication 1, dans laquelle $R_1$, $R_2$, $R_3$ et $R_4$ ont les significations indiquées ci-dessus, avec l'anhydride sulfurique ou un composé fournissant un reste de formule partielle Ia ou Ib de la revendication 1, ou bien b), dans un composé de formule III dans laquelle $R_1$, $R_2$, $R_3$ et $R_4$ ont les significations indiquées ci-dessus et $A_o$ représente un reste de formule IIIa ou IIIb dans laquelle $Y_1$, $Y_2$ et $Y_3$ ont les significations indiquées dans la revendication 1, on remplace le reste $Y_1$ ou respectivement le reste $Y_3$ et le cas échéant le reste $Y_2$ par le groupe $O-Z_1$ ou $O-Z_3$ respectivement et le cas échéant $O-Z_2$ éventuellement à l'état de sels, et si on le désire, on convertit un produit de réaction de formule générale I dans laquelle $Z_1$ ou $Z_3$ et le cas échéant $Z_2$ représentent l'hydrogène, en un sel d'une base ou bien, à partir d'un sel obtenu, on libère l'acide correspondant et si on le désire, on convertit un composé de formule générale I, de manière connue en soi, en un autre composé de formule générale I ou en un sel d'un tel composé.

3. Procédé selon la revendication 1, caractérisé en ce que l'on utilise en tant que composé introduisant le reste de formule partielle Ia l'anhydride sulfurique ou un complexe de cet anhydride.

4. Procédé selon la revendication 1, caractérisé en ce que l'on utilise en tant que composé introduisant le reste de formule Ia ou Ib respectivement un composé de formule respective

$X_1-SO_2-O-Z_1$ et 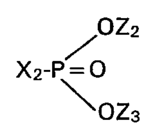

dans laquelle $X_1$ et $X_2$ représentent des groupes

hydroxy ayant subi une modification fonctionnelle et réactifs.

5. Procédé selon la revendication 2, caractérisé en ce que l'on utilise en tant que composé introduisant le reste de formule Ia ou Ib un composé de formule respective

$X_1-SO_2-O-Z_1$ et 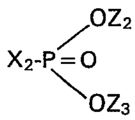

dans laquelle $X_1$ et $X_2$ représentent des groupes hydroxy ayant subi une modification fonctionnelle et réactifs.

6. Procédé selon la revendication 1, caractérisé en ce que $Y_1$ ou $Y_2$ et/ou $Y_3$ représentent des groupes hydroxy estérifiés ou éthérifiés.

7. Procédé selon la revendication 2, caractérisé en ce que $Y_1$ ou $Y_2$ et/ou $Y_3$ représentent des groupes hydroxy estérifiés ou éthérifiés.

8. Procédé selon la revendication 6, caractérisé en ce que l'on remplace un groupe hydroxy éthérifié par un groupe hydroxy par une réaction de substitution nucléophile.

9. Procédé selon la revendication 8, caractérisé en ce que l'on utilise un réactif nucléophile approprié contenant un groupe hydroxy ou mercapto éthérifiable ou un groupe amino substituable, y compris quaternisable.

10. Procédé selon la revendication 7, caractérisé en ce que l'on remplace un groupe hydroxy éthérifié par un groupe hydroxy en traitant par un thiophénate.

11. Procédé selon l'une des revendications 1, 3, 4, 6, 8 et 9, caractérisé en ce que l'on prépare des composés de formule I selon la revendication 1, dans laquelle l'un des symboles $R_1$ et $R_2$ représente un groupe allyle ou 2-méthylallyle et l'autre représente aussi un tel groupe ou bien un groupe méthyle, $R_3$ et $R_4$ ont les significations indiquées dans la revendication 1 et A représente un reste de formule partielle Ia dans laquelle $Z_1$ représente l'hydrogène ou un reste de formule partielle Ib dans laquelle $Z_2$ représente un groupe alkyle inférieur contenant jusqu'à 4 atomes de carbone, et $Z_3$ représente un groupe alkyle inférieur contenant jusqu'à 4 atomes de carbone ou l'hydrogène, et des sels de ces composés pour lesquels $Z_1$ ou $Z_3$ et le cas échéant également $Z_2$ représentent l'hydrogène.

12. Procédé selon l'une des revendications 2, 5, 7 et 10, caractérisé en ce que l'on prépare des composés de formule I selon la revendication 1 dans laquelle l'un des symboles $R_1$ et $R_2$ représente un groupe allyle ou 2-méthylallyle et l'autre représente également l'un de ces groupes ou bien un groupe méthyle, $R_3$ et $R_4$ ont les significations indiquées dans la revendication 1 et A représente un reste de formule partielle Ia dans laquelle $Z_1$ représente l'hydrogène ou un reste de formule partielle Ib dans laquelle $Z_2$ représente un groupe alkyle inférieur contenant jusqu'à 4 atomes de carbone, et $Z_3$ représente un groupe alkyle inférieur contenant jusqu'à 4 atomes de carbone ou l'hydrogène, et des sels de ces composés pour lesquels $Z_1$ ou $Z_3$ et le cas échéant également $Z_2$ représentent l'hydrogène.

13. Procédé selon l'une des revendications 1, 3, 4, 6, 8 et 9, caractérisé en ce que l'on prépare le méthyl-[3-méthyl-2-{[5-méthyl-3-(2-méthylallyl)-4--oxo-2-thiazolidinylidène]-hydrazono}-4-oxo-5-thiazolidinyl]-hydrogénophosphate et ses sels.

14. Procédé selon l'une des revendications 2, 5, 7 et 10, caractérisé en ce que l'on prépare le méthyl-[3-méthyl-2-{[5-méthyl-3-(2-méthylallyl)-4--oxo-2-thiazolidinylidène]-hydrazono}-4-oxo-5-thiazolidinyl]-hydrogénophosphate et ses sels.